(19) Europäisches Patentamt
European Patent Office
Office européen des brevets

(11) Publication number: **0 593 765 A1**

# (12) EUROPEAN PATENT APPLICATION published in accordance with Art. 158(3) EPC

(21) Application number: **91920691.2**

(22) Date of filing: **27.11.91**

(86) International application number:
**PCT/JP91/01628**

(87) International publication number:
**WO 93/11139 (10.06.93 93/14)**

(51) Int. Cl.5: **C07F 9/655**, C07H 13/06, //A61K31/70,A61K31/665

(43) Date of publication of application:
**27.04.94 Bulletin 94/17**

(84) Designated Contracting States:
**AT BE CH DE DK ES FR GB GR IT LI LU NL SE**

(71) Applicant: **JAPAN TOBACCO INC.**
**12-16, Higashi Shinagawa 4-chome,**
**Shinagawa-ku, Tokyo 140(JP)**
Applicant: **HASEGAWA, Akira**
**1735-160, Ohkurayama, Kano**
**Gifu-shi, Gifu-ken 501-31(JP)**

(72) Inventor: **HASEGAWA, Akira**
**1735-160, Ohkurayama**
**Kano**
**Gifu-shi Gifu-ken 501-31(JP)**
Inventor: **KISO, Makoto**
**57-47, Monju**
**Motosucho**
**Motosugun**
**Gifu-ken 501-12(JP)**
Inventor: **UESATO, Shinichi Pharmac. Res. Labs of**
**Japan Tobacco Inc.**
**6-2, Umegaoka**
**Midori-ku**
**Yokohama-shi Kanagawa-ken 227(JP)**
Inventor: **ISHIDA, Tomio Pharmac. Res. Labs of**
**Japan Tobacco Inc.**
**6-2, Umegaoka**
**Midori-ku**
**Yokohama-shi Kanagawa-ken 227(JP)**
Inventor: **SUZUKI, Masanobu Pharmac. Res. Labs of**
**Japan Tobacco Inc.**
**6-2, Umegaoka**
**Midori-ku**
**Yokohama-shi Kanagawa-ken 227(JP)**
Inventor: **SAITO, Yutaka Pharmac. Res. Labs of**
**Japan Tobacco Inc.**
**6-2, Umegaoka**
**Midori-ku**
**Yokohama-shi Kanagawa-ken 227(JP)**

(74) Representative: **Reinhard, Horst, Dr. et al**
**Patentanwälte**
**REINHARD . SKUHRA . WEISE,**
**Postfach 44 01 51**
**D-80750 München (DE)**

## (54) 6-SUBSTITUTED LIPID A ANALOG.

(57) A 6-substituted lipid A analog represented by general formula [I] wherein $R_1$ represents hydrogen or hydroxy; $R_2$ represents $-C(O)R_3$; $R_3$ represents linear or branched lower alkyl which may be substituted with carboxy or lower alkoxycarbonyl; l represents an integer of 5 to 17; m represents an integer of 7 to 19; n represents an integer of 4 to 16; and p represents 0 or 1.

EP 0 593 765 A1

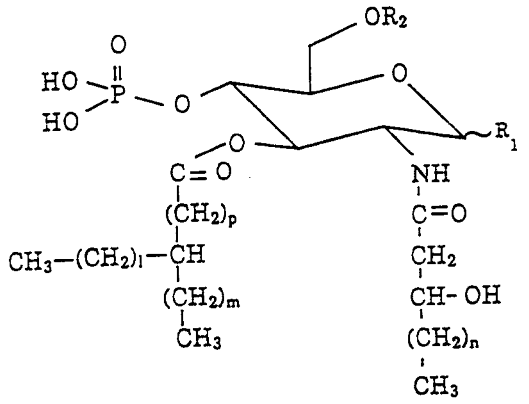

[1]

[Technical Field]

This invention relates to novel 6-substituted lipid A analogues which show lipid A-like activity useful as pharmaceutical drugs such as immunopotentiating agent and antitumor agent.

[BACKGROUND ART]

Surface layers of Gram-negative bacteria are composed of cell membranes, cell wall peptide glucan surrounding the membranes, and outer membranes. The outer membrane contains lipopolysaccharide (hereinafter called LPS) which is a main ingredient of endotoxin that induces endotoxin shock.

LPS consists of three components, an acidic protein component, a high molecular polysaccharide component, and a phospholipid component, and it shows such functions as causing pyretogenesis, hemorrhage, arthritis, and encephalomyelitis, and has been also known to show immunopotentiating effect of a host-protecting mechanism (macrophage activation, B-cell blastogenesis, cell-mediated immunity activation, etc.), as well as antitumor effect (interferon induction, tumor necrosis factor induction, etc.).

LPS expressed its activity mainly by the phospholipid part called lipid A among said three components, and it comprises fatty acid residue and phosphoric acid both of which are combined with disaccharide amine, and has the following formula [Japanese Bacteriology Journal 40 (1), 57 (1985) and Proc. Natl. Acad. Sci. USA 80, 4624 (1983)].

Lapid A of E.coli.

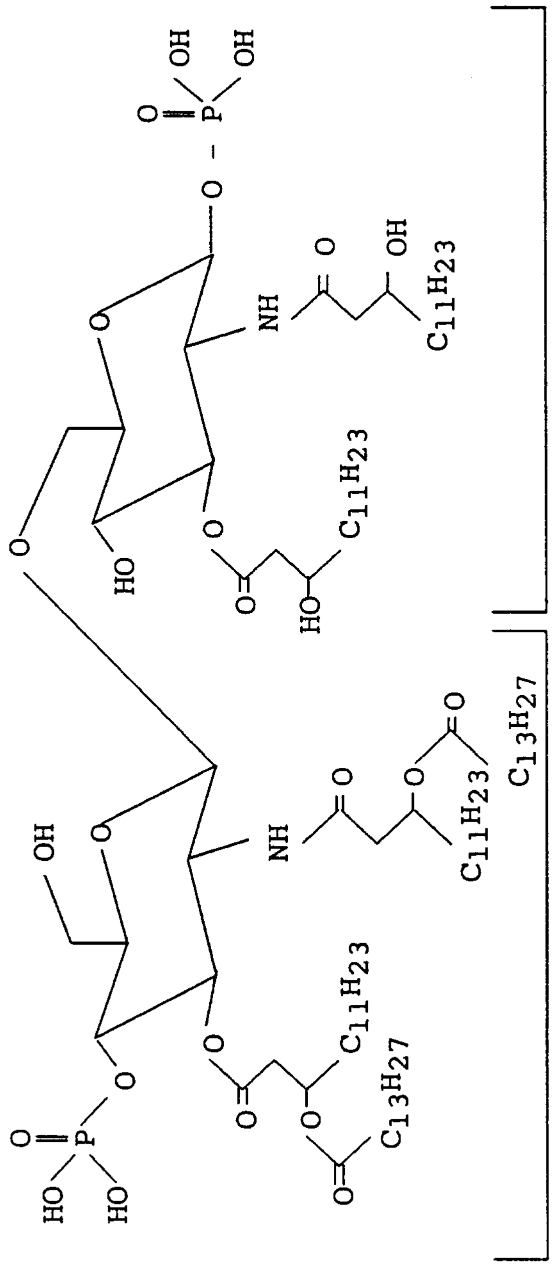

Reducing Subunit

Non-reducing subunit

Recent studies have revealed that either a non-reducing or a reducing subunit as shown above alone possesses lipid A-like activity, and various analogues have been synthesized.

For example, the Japanese Patent Disclosure No. 501259/85 disclosed lipid A analogues which closely approximate to the reducing subunit and a method for producing the analogues, the Japanese Patent Disclosure No. 146891/89 discloses monophosphoryl lipid A derivatives, the Japanese Patent Disclosure No. 246195/86 discloses novel disaccharide and trisaccharide derivatives of lipid A type, the Japanese Patent Disclosure Nos. 52793/89 and 179885/88 disclose glucopyranose derivatives obtained by converting a phosphoric acid residue of the non-reducing subunit in lipid A into sulfuric acid residue, the Japanese Patent Disclosure No. 129293/87 discloses glucopyranose derivatives introducing succinic acid or methyl succinate in 6-position of the non-reducing subunit, and the Japanese Patent Disclosure No. 256697/90 discloses lipid A monosaccharide analogues having 6-position of the non-reducing subunit protected with aliphatic acyl group.

The present inventors have attempted the various syntheses in order to obtain lipid A derivatives having a stronger activity, and synthesized numerous novel derivatives, in particular by changing the substituents or substituent sites of non-reducing subunit derivatives, and as a result discovered that a potent activity

similar to that of natural lipid A is noted among such derivatives, and file so far many patent applications (e.g., the Japanese Patent application Nos. 95024/90, 44365/90, and 241866/89, and Japanese Patent Disclosure Nos. 62888/90, 62889/90, 25494/90, 146892/89, 44588/88, 33391/88, 30495/88, 129292/87, 172867/86, 126094/86, and 126093/86).

As described above, extensive studies have been conducted on lipid A analogues, and modifications with various substitutes and substituent sites have been investigated in detail. However, such a problem has not yet overcome as obtaining different activities for different introduction sites of the same substituents, and nothing applicable as pharmaceutical agents has been developed; therefore, compounds showing stronger activities and lower in toxicity are keenly demanded.

[DISCLOSURE OF THE INVENTION]

The present inventors intensively continued studies on lipid A derivatives in order to solve the above problems, and discovered compounds showing strong activities similar to those of natural lipid A, such as, although somewhat different in potency, mitogen activity, tumor necrosis factor induction, and interferon induction, as well as low in toxicity, and finally completed the present invention.

According to the invention, novel 6-substituted lipid A analogues have the structure expressed in the following formula [I]:

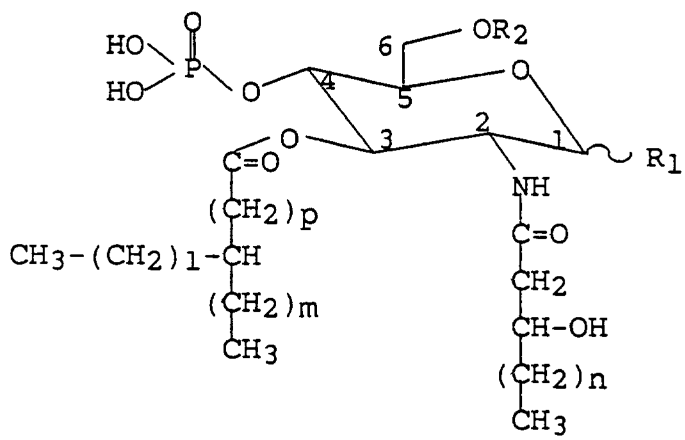

[I]

wherein $R_1$ represents a hydrogen atom or hydroxy group, $R_2$ indicates $-C(O)R_3$, $R_3$ denotes a lower alkyl group of straight chain or branch which may be substituted by carboxy group or lower alkoxycarbonyl group, 1 is an integer of 5 to 17, m is an integer of 7 to 19, n is an integer of 4 to 16, and p is either 0 or 1.

Here, the lower alkyl group indicates an alkyl group containing 1 to 5 carbon atoms, or preferably 1 to 4.

Moreover, $R_3$ represents either straight chain alkyl group such as methyl, ethyl, propyl, butyl or pentyl, or branch alkyl group such as isopropyl, isobutyl or tert-butyl, and these alkyl groups may be substituted by carboxyl group, or lower alkoxycarbonyl group such as methoxycarbonyl or ethoxycarbonyl.

The 6-substituted lipid A analogue [I] of the invention is characterized in the structure in that the 3-position of the ring is acylated by $\alpha$- or $\beta$-alkyl substituted fatty acid, and that a substituent is introduced in the 6-position, and its utility as a pharmaceutical agent is particularly expected. In these compounds [I], stereoisomers can exist, but its isolated isomers and a mixture of them are all included in the invention.

These 6-substituted lipid A analogues [I] may be produced, for example, by the following reactions.

FLOW 1

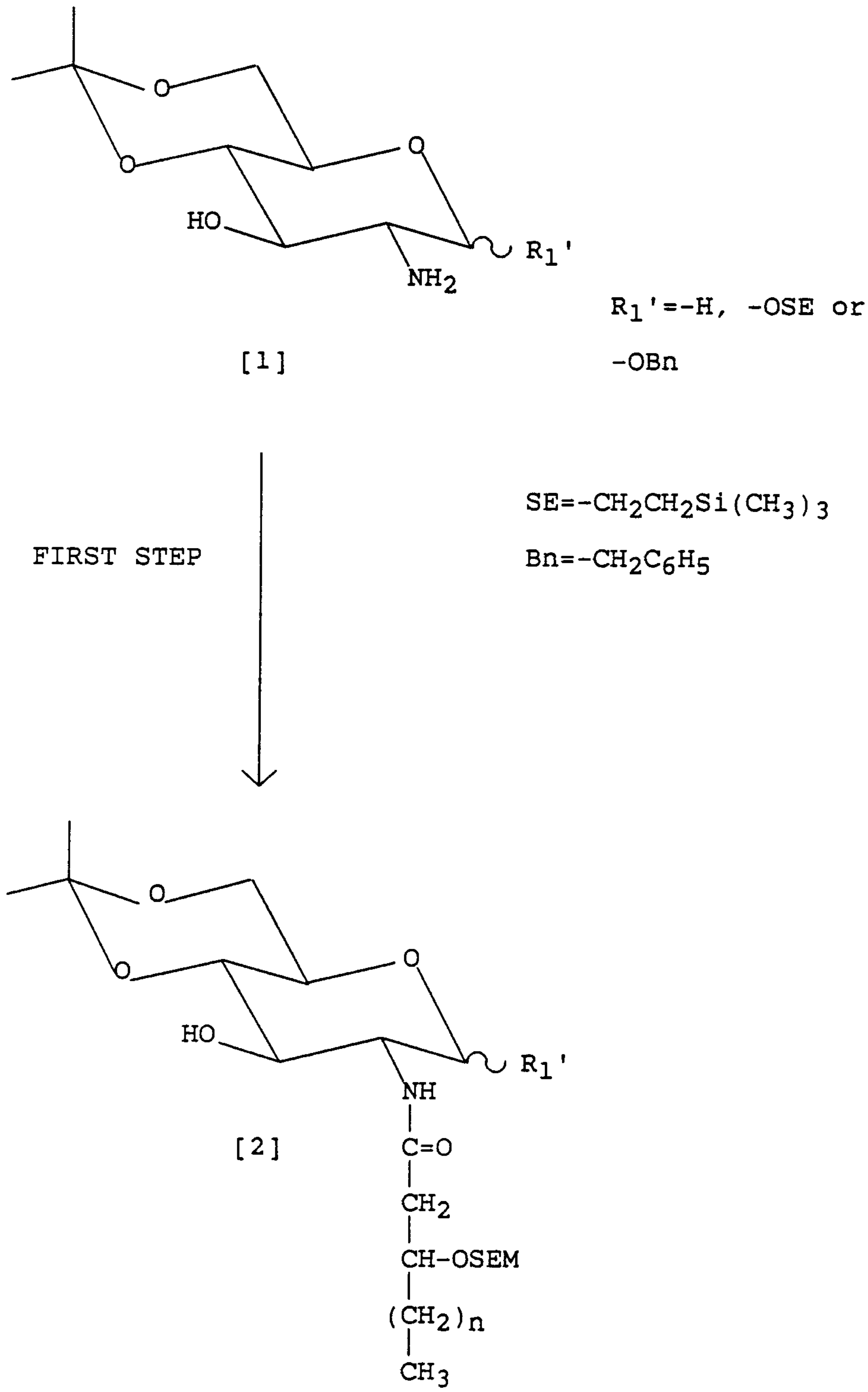


[1]

[2]

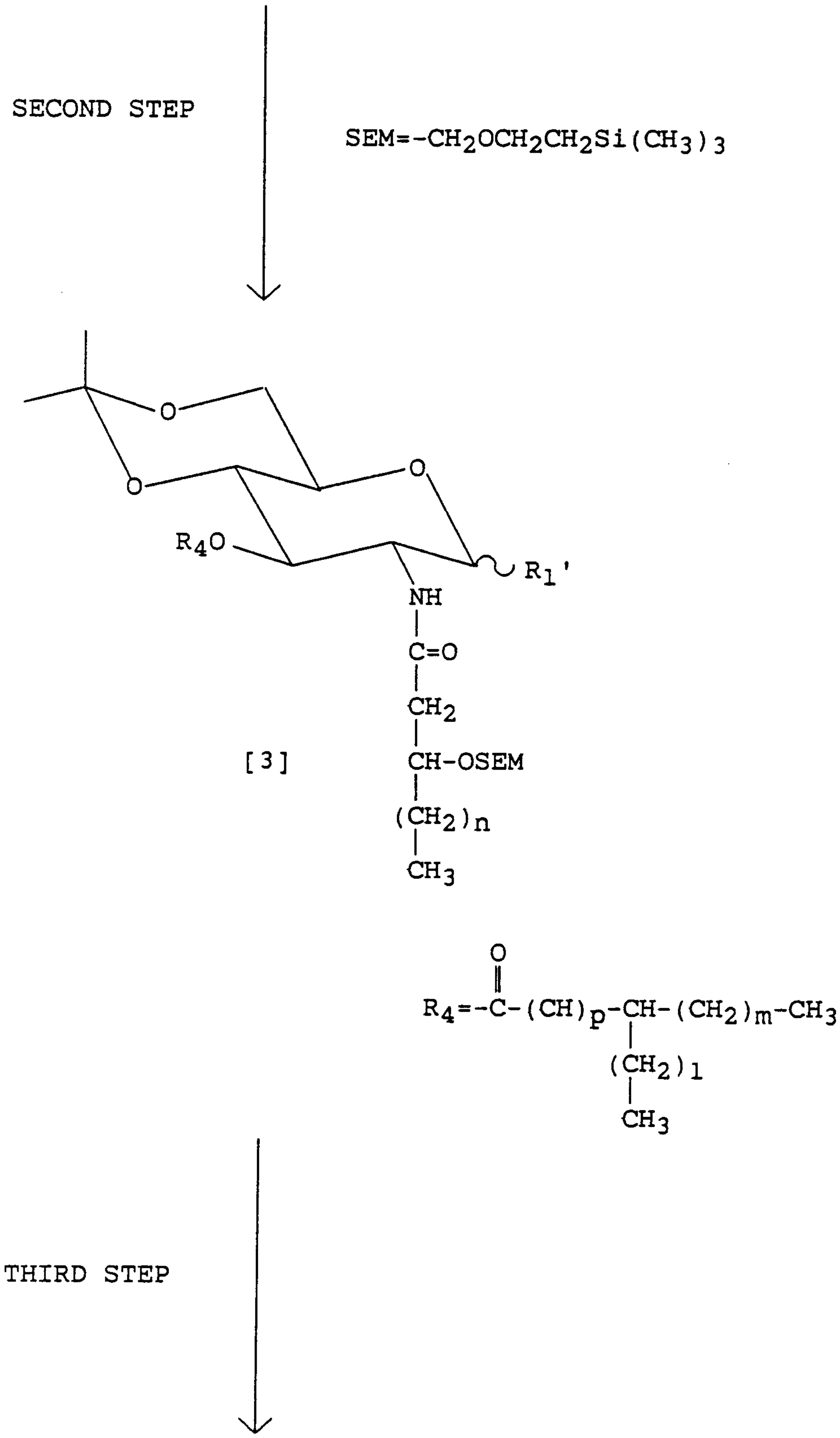


[3]

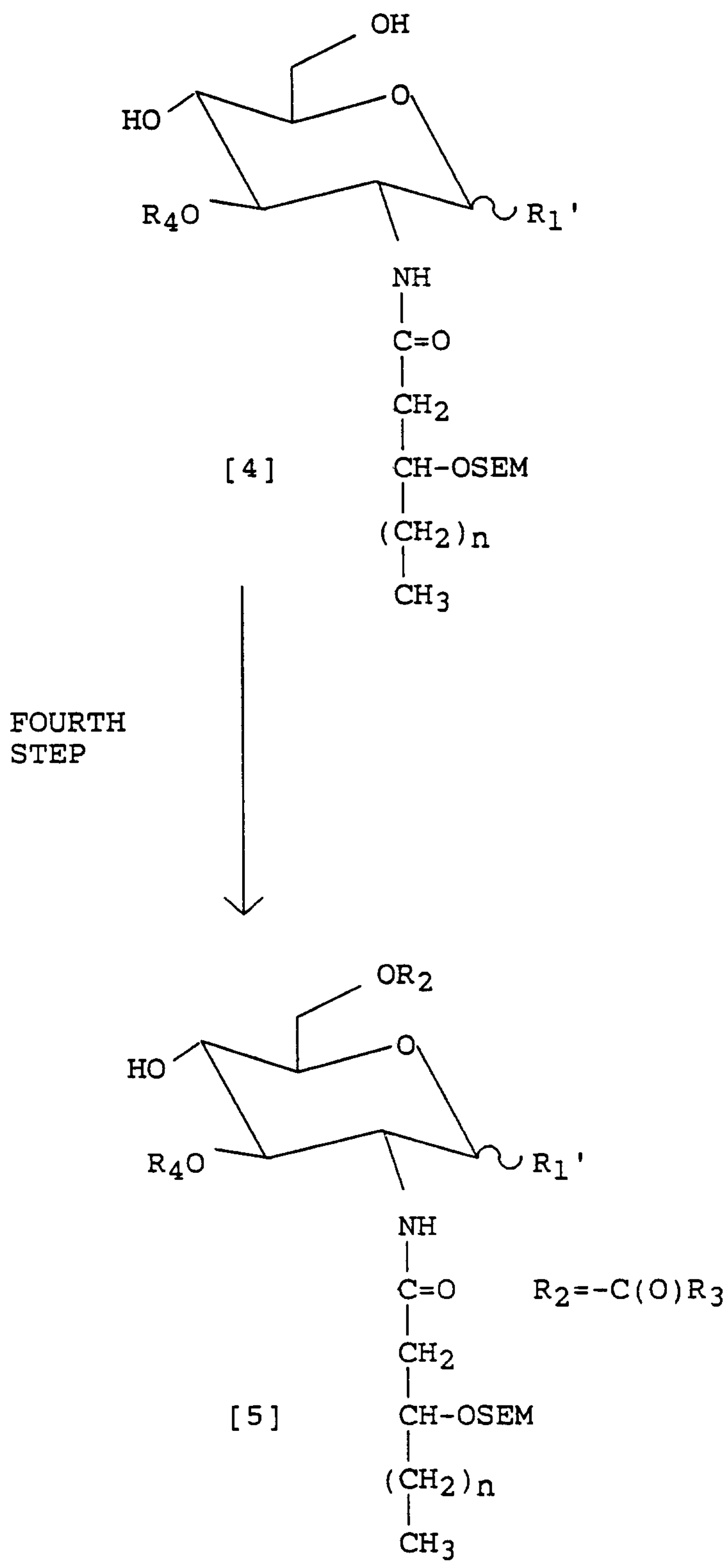


[4]

[5]

$R_2$=-C(O)$R_3$

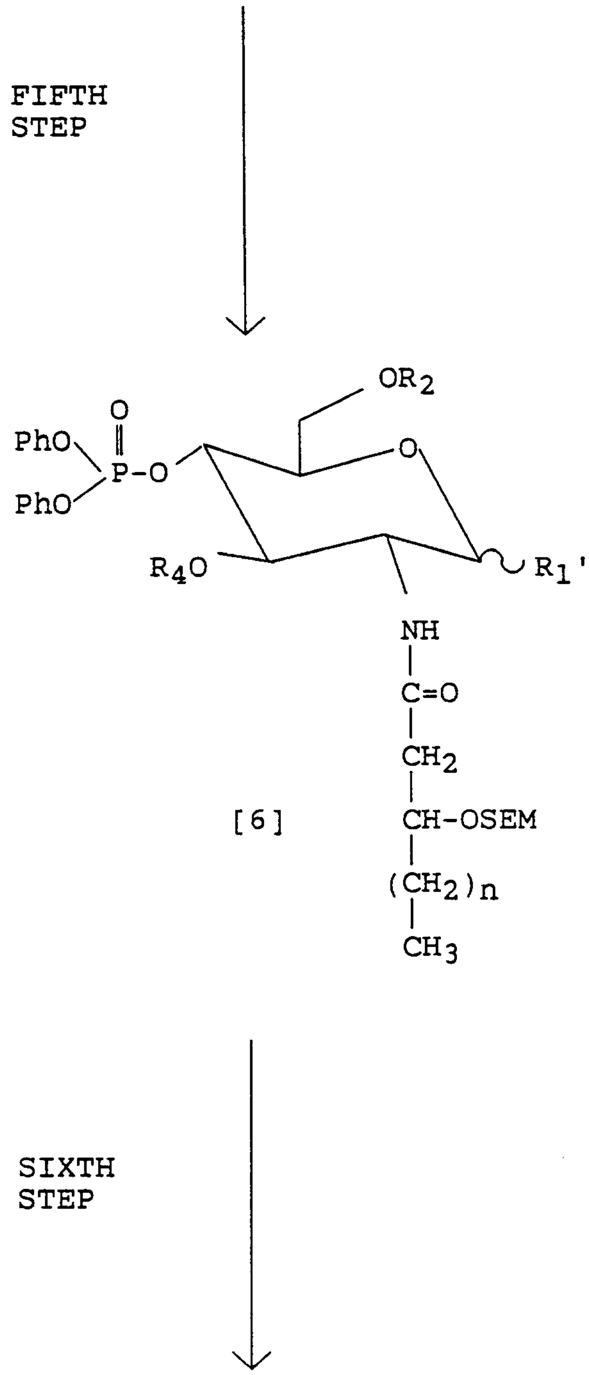


[6]

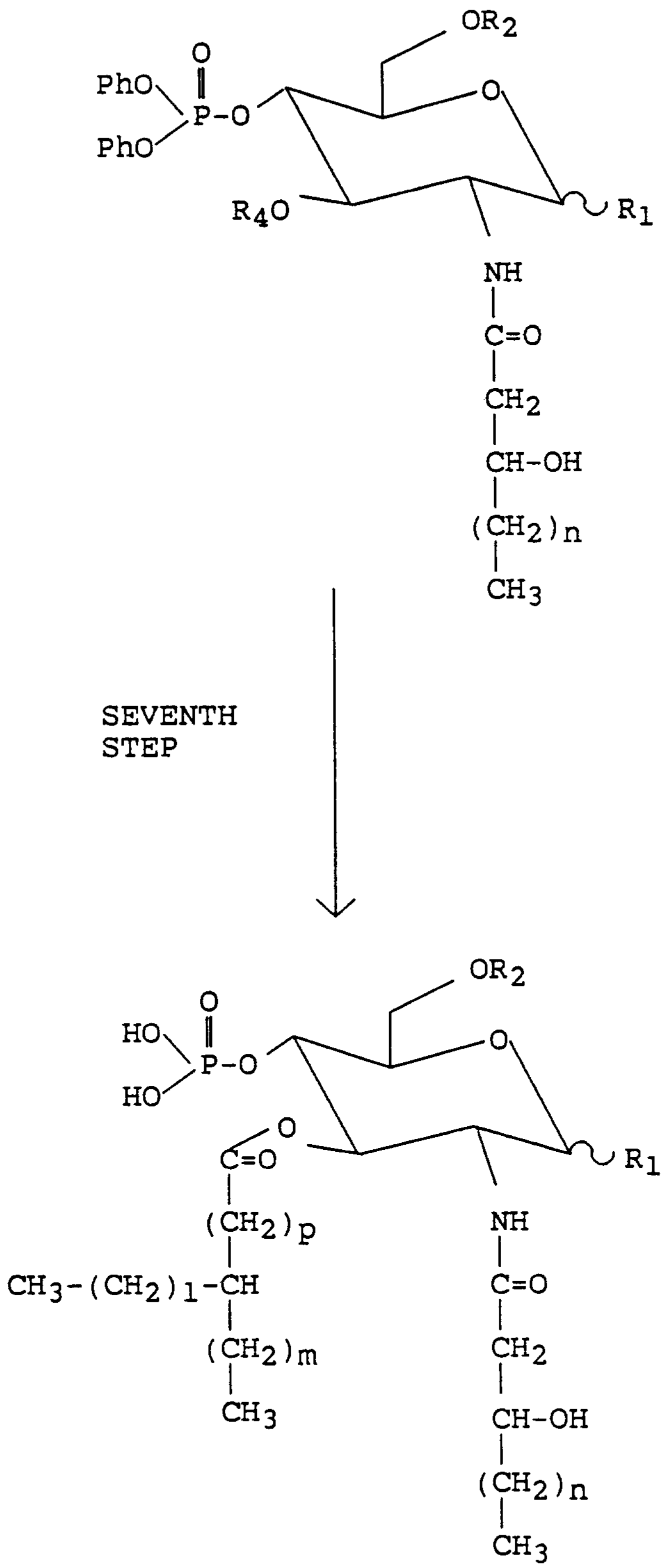


[7]

[I]

(First step)

A known compound [1] derived from D-glucosamine (e.g. see Japanese Patent Disclosure No. 197582/86) is amidated by reaction with a fatty acid compound, 3-position of which possesses hydroxyl group protected with 2-(trimethylsilyl)ethoxymethyl group (SEM group), in the presence of condensation

agent such as 1-ethyl-3-(3-dimethylaminopropyl)carbodiimide hydrochloride (WSC•HCℓ) or dicyclohexylcarbodiimide (DCC) in an inert solvent such as dichloromethane to yield a compound [2].

(Second step)

A compound expressed in a formula [2] possessing a hydroxyl group at other than position 3 properly protected, such as 1,5-anhydro-2-deoxy-4,6-O-isopropylidene-2-{(3R)-3-[2-(trimethylsilyl)-ethoxymethoxy]-tetradecanamido}-D-glucitol is added with a catalytic amount of dimethylaminopyridine (DMAP) in an inert solvent such as dichloromethane, and the mixture is reacted with $\alpha$- or $\beta$-alkyl substituted fatty acid ($R_4OH$) in the presence of condensation agent such as 1-ethyl-3-(3-dimethylaminopropyl)carbodiimide hydrochloride (WSC•HCℓ) or dicyclohexylcarbodiimide (DCC) to acylate the hydroxyl group at position 3 of the ring, thereby yielding a compound [3].

(Third step)

The compound [3] obtained in the second step is hydrolyzed with an acid such as aqueous acetic acid to eliminate the protecting groups at 4, 6-positions, thereby yielding a compound [4].

(Fourth step)

The compound [4] dissolved in an inert solvent such as pyridine or dichloromethane is reacted with acyl halide ($R_2$-X; X indicating halogen) such as pivaloyl chloride or 3-(methoxycarbonyl)propionyl chloride, or with an acid anhydride such as succinic anhydride in the presence of a base to give a compound [5] possessing an acyl group at 6-position.

In another way, the compound [5] possessing an acyl group at 6-position may also be obtained by treatment of compound [4] with monoester of dicarboxylic acid such as adipinic acid monoethyl ester or carboxylic acid in an inert solvent such as dichlorometane containing a catalytic amount of dimethylaminopyridine (DMAP) in the presence of 1-ethyl-3-(2-dimetylaminopropyl)carbodiimide hydrochloride (WSC•HCℓ) or dicyclohexylcarbodiimide (DCC).

(Fifth step)

The compound [5] is dissolved in pyridine, dichloromethane, their mixed solvent etc. together with DMAP and then treated with diphenylphosphono chloride to yield a compound [6] possessing diphenyl phosphono group at 4-position.

(Sixth step)

An acid such as boron trifluoride etherate ($BF_3 \cdot OEt_2$), or fluoride ion generating agent such as tetrabutylammonium fluoride is added to the compound [6] in a solvent such as dichloromethane. By this reaction, protecting group at 3-position of fatty acid residue which is bonded to 2-position of the ring is eliminated to yield a compound [7].

When an object compound of which $R_2$ is lower alkyl group substituted with carboxyl group is prepared, the carboxyl group may be protected with benzyl group (-Bn group) during the steps 4-6, and the protecting group may be eliminated just before the seven step.

(Seventh step)

The compound [7] is hydrogenated over platinum oxide ($PtO_2$) in a solvent such as ethanol or methanol to afford the final object compound [I] possessing a phosphono group at 4-position.

In another way, compound [6] may be also obtained by, after the third step, substitution of the 4-position of the ring with diphenylphosphono group, and then substitution of the 6-position with acyl group. These procedures may be carried out according to the following Flow 2.

FLOW 2

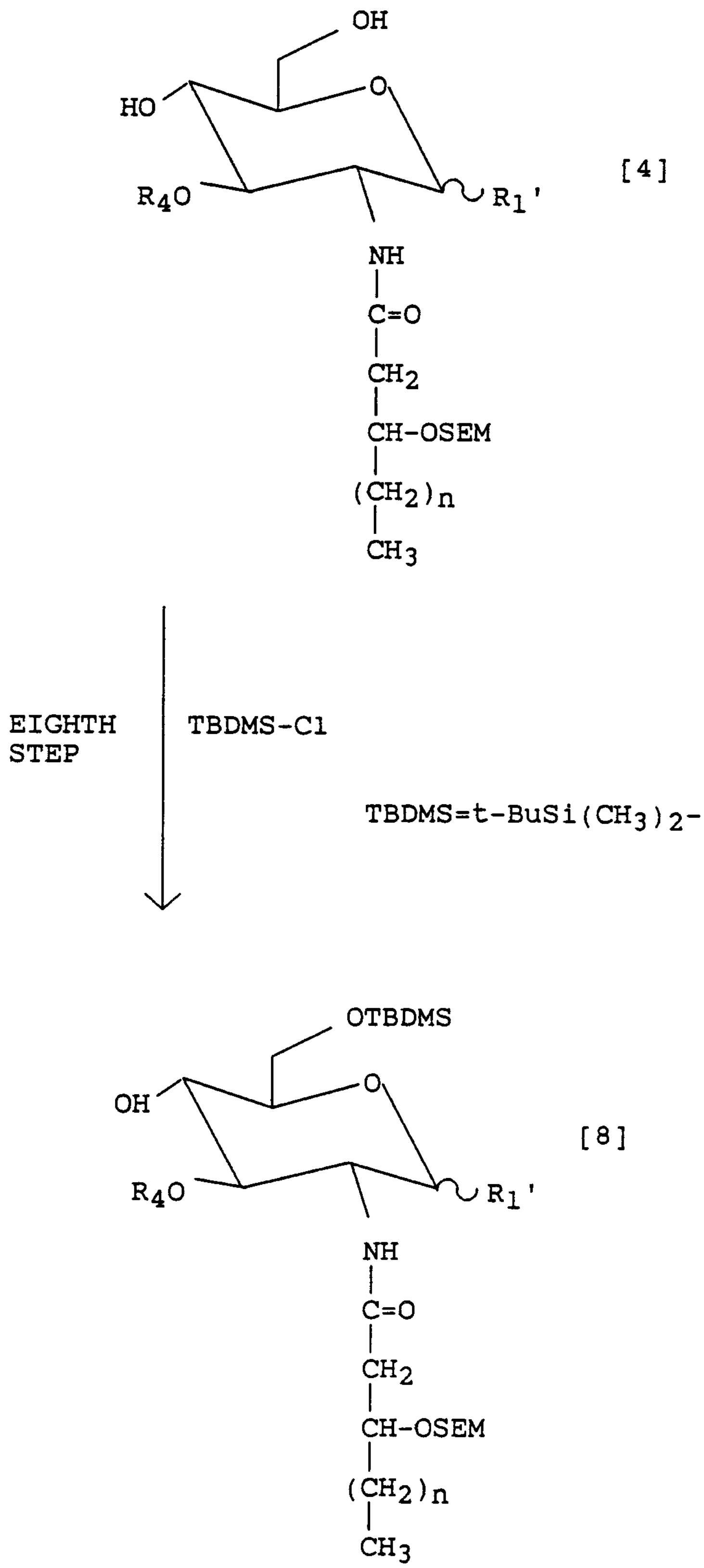


[4]

[8]

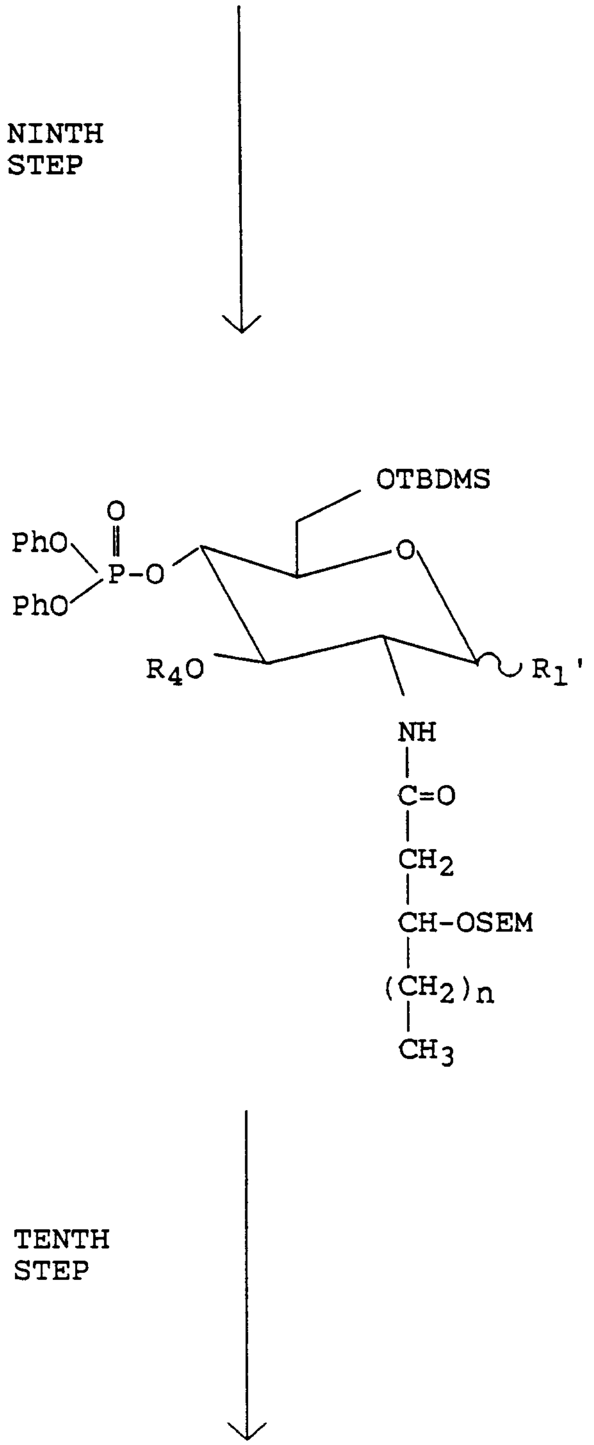


[9]

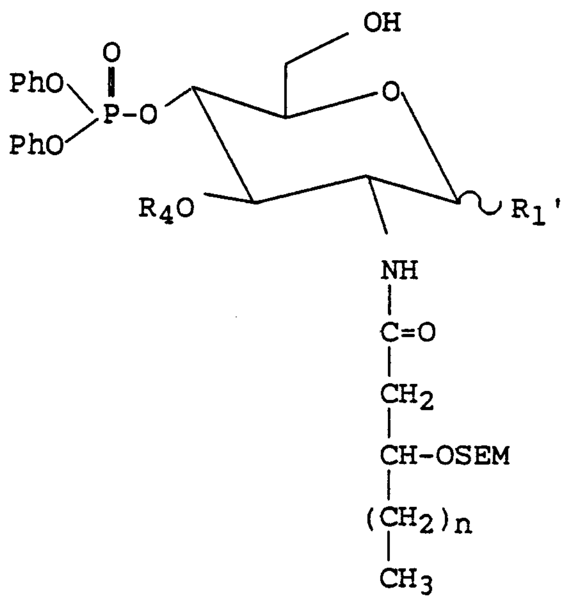

[10]

ElEVENTH STEP

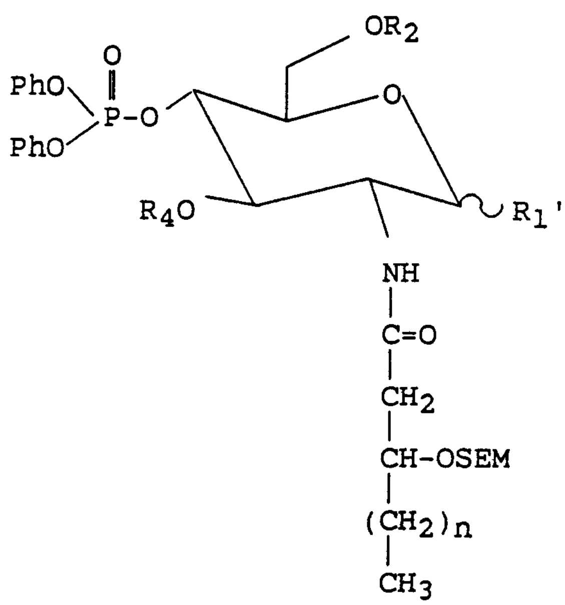

[6]

(Eighth step)

The compound [4] obtained in the third step is dissolved in an inert solvent such as pyridine or dichloromethane, and is reacted with tert-butyldimethylsilyl chloride (TBDMS-Cℓ) to afford a compound [8]

protected with TBDMS at 6-position.

(Ninth step)

The compound [8] is dissolved in pyridine, dichloromethane, their mixed solvent, etc. together with DMAP, and is reacted with diphenyl phosphono chloride to afford a compound [9] possessing a dipheynlphosphono group at 4-position.

(Tenth step)

The compound [9] obtained in the ninth step is eliminated the protecting group at 6-position by hydrolysis with an acid such as aqueous acetic acid to give a compound [10].

(Eleventh step)

The compound [10] is treated in the same method as the fourth step in Flow-1 to obtain the compound [6] possessing an acyl group at 6-position. Afterwards, going to the sixth step in Flow-1, the final compound [I] can be obtained.

In the reaction Flows-1, -2, in order to prepare derivatives of which 1-position is deoxy, that is, $R_1$ is hydrogen, the reactions of the steps 1-7 may be carried out in the state of $R_1$ = -H in the same as described above. On the other hand, if desiring the final object compound [I] of which $R_1$ is a hydroxyl group, the hydroxyl group should be protected with trimethylsilylethyl group (-SE group), benzyl group (-Bn group) or the like from the first step to the fifth step or the eleventh step to remain in a form of $R_1$ = -OSE or -OBn, and this protecting group should be eliminated at the sixth step by an ordinary method.

Some of the $\alpha$-alkyl substituted fatty acids used in the second step are known. Others may be prepared easily from known compounds. For example, in a mixed solvent of anhydrous tetrahydrofuran and anhydrous hexamethylphosphoryltriamide (HMPA), 2 equivalent of lithium diisopropylamide is added to the straight-chain carboxylic acid having a corresponding number of carbon atoms to produce dianion. Then straight-chain alkyl halide having a corresponding number of carbon atoms is added to the dianion, thereby reacting each others, so that an alkyl side chain may be introduced into $\alpha$-position of carboxylic acid. It may be expressed as follows.

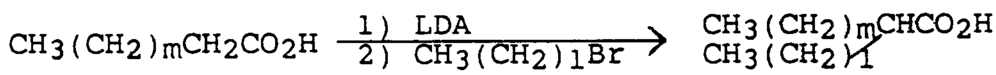


Besides, the $\beta$-alkyl substituted fatty acid in the second step may be prepared in the following reactions.

FLOW 3

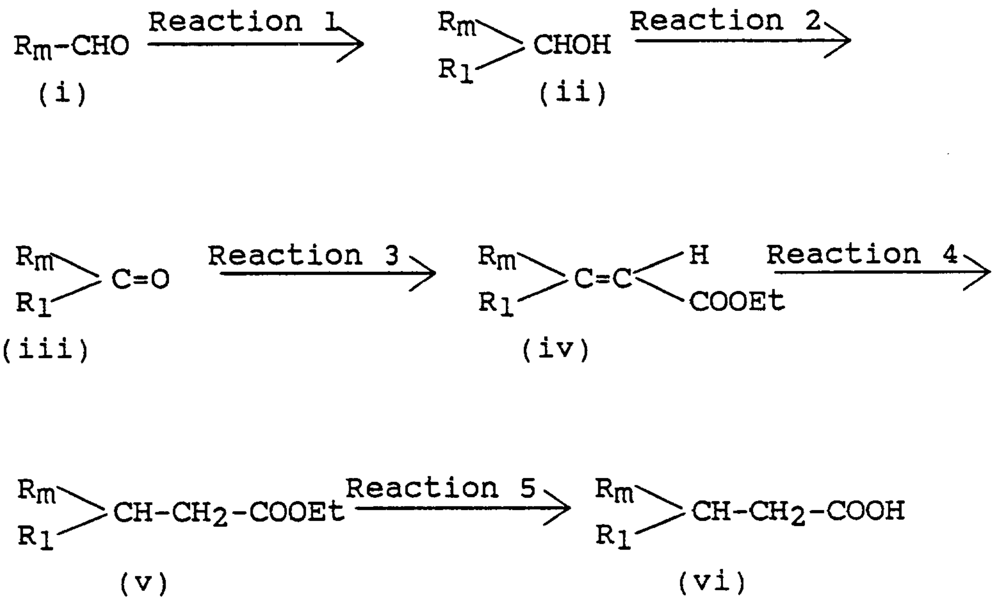


in which Rm represents $H_3C(CH_2)_m$-, and $R_1$ denotes $H_3C(CH_2)_1$-.

(Reaction 1)

1-Halogenated alkyl is reacted with metal magnesium in an anhydrous solvent to prepare a Grignard reagent of $R_1$-MgBr type, and it is reacted with aldehyde of formula (i) to yield a secondary alcohol of formula (ii).

(Reaction 2)

Using pyridinium chlorochromate (PCC) as an oxidizing agent, the compound (ii) is oxidized in dried methylene chloride to yield a ketocompound (iii).

(Reaction 3)

This is a so-called Witting reaction. Sodium hydride is added to a dried benzene containing triethyl phosphonoacetate, stirred, and then the compound (iii) is added to the mixture, thereby reacting each others. By this reaction, a compound (iv) is obtained.

(Reaction 4)

The compound (iv) dissolved in a solvent is hydrogenated in the presence of a catalyst such as palladium carbon to yield an ester compound (v).

(Reaction 5)

The ester compound (v) is hydrolyzed with alkali or the like to yield the objective β-alkyl substituted fatty acid (vi).

⟨Applications to Pharmaceutical Drugs⟩

The compound of the invention expressed in Formula [I] is generally administered systemically or topically, and orally or parenterally.

The dose varies with the age, body weight, symptom, therapeutic effect, route of administration, treatment period, etc., but generally a single dose of 0.01 mg to 100 mg for an adult is administered once to several times a day either orally or parenterally.

The solid composition for oral administration of the invention includes tablets, powder, and granules, among others. In such solid composition, one or more active substances are mixed with at least one inert diluent or dispersing agent, such as lactose, mannitol, glucose, hydroxypropylcellulose, crystalline cellulose, starch, polyvinylpyrrolidone, magnesium aluminometasilicate, or absorbent such as anhydrous silica powder. The composition may also contain, according to a conventional method, additives other than inert diluents.

Tablets or pills may be coated with, if desired, gastric soluble or enteric soluble films such as saccharose, gelatin, hydroxypropylcellulose, and hydroxypropylmethylcellulose phthalate, or with two or more layers of them. The composition may be also contained in capsules of gelatin, ethylcellulose or similar substance.

The liquid composition for oral administration may include pharmaceutical acceptable emulsion, solution, suspension, syrup, elixir, and others, and may also include generally employed inert diluents, such as purified water, ethanol, vegetable oil, and emulsifying agent. Such composition may contain adjuvants such as infiltrating agent and suspending agent, sweeteners, flavoring agents, perfumes, and antiseptics, aside from the inert diluents. The injection for parenteral administration may contain sterile aqueous or non-aqueous solvents, solubilizing agents, suspending agents, and emulsifying agents. Examples of aqueous solvents, solubilizing agents, and suspending agents include, among others, distilled water for injection, saline, cyclodextrin and its derivatives, organic amines such as triethanolamine, diethanolamine, mono-oethanolamine and triethylamine, and inorganic alkalines.

Examples of non-aqueous solvents include, for example, propyleneglycol, polyethyleneglycol, vegetable oils such as olive oil, and alcohols such as ethanol. Examples of non-aqeuous solubilizing agents include, for example, surfactants (which form mixed micelles) such as polyoxyethylene hydrogenated castor oil and sucrose fatty acid ester, lecithin, and hydrogenated lecithin (which forms liposomes), etc. Examples may also include emulsion preparations, which are obtained by using non-aqueous solvents such as vegetable oils with emulsifying agents such as lecithin, polyoxyethylene hydrogenated castor oils, and polyoxyethylenepolyoxypropylene glycol.

Other compositions for parenteral administration include topical solutions, liniments such as ointments, suppositories, and pessaries, which contain one or more active substances and are prescribed according to the known methods.

Hereinafter are described pharmacological actions of the compounds of the invention by way of experimental examples, and aside from the disclosed experiments the compounds of the invention also produced significant results in immunopotentiating experiments such as colony stimulating factor induction, and also showed toxicities for various tests including local Schwartzman reaction and pyrogenicity.

Experiment 1.

$O_2^-$ production stimulating activity in neutrophils

The $O_2^-$ production stimulating activity in neutrophils was evaluated in the following experimental system [see J. Exp. Med., Vol. 160, 1656 - 1671, 1984].

In the peritoneal cavity of C3H/HeN mouse (male, 8 - 9 weeks old), physiological saline containing 0.2% (w/v) of casein was administered. Three hours later, peritoneal exudate cells (90% or more of which are neutrophils) induced in the peritoneal cavity were collected. These cells ($1.7 \times 10^6$ cells/ml/tube) were incubated in the presence of the compound of the invention (10 $\mu$g/m$\ell$) at 37 °C for 60 minutes, and cytochrome C (80 $\mu$M) and fomyl-methionyl-leucyl-phenylalanine (FMLP) (0.1 $\mu$M) were added. After reaction for 10 minutes at 37 °C in the presence of or in the absence of superoxide dismutase (SOD), the SOD-inhibitable cytochrome C reduction quantity was calculated from the difference of absorbances at 550 nm and 541.7 nm and the molar absorption coefficient ($16.5 \times 10^3$). The $O_2^-$ production stimulating activity was expressed in the following stimulation percentage.

$$\text{Stimulation (\%)} = \frac{O_2^- \text{ production in the presence of the compound of the invention}}{O_2^- \text{ production in the absence of the invention}} \times 100-100$$

The compounds of the invention presented the activities as shown in the below table.

Table 1

| Compound | Stimulation percentage |
|---|---|
| No compound | 0 |
| Control compound | 80 |
| Example 1 | 363 |
| Example 4 | 293 |

Control compound: 1,5-Anhydro-2-deoxy-3-O-[(2RS)-2-dodecylhexadecanoyl]-2-[(3R)-3-hydroxytetradecanamido]-4]-O-phosphono-D-glycitol [corresponding to nonsubstituted compounds A, D ($R_2$ = H) at 6-position]

Experiment 2.

TNF producing activity

The producing activity of TNF was evaluated in the following experiment.

The first stimulating agent, 5% suspension (0.2 mℓ physiological saline) of Corynebacterium parvum was intravenously administered to ICR mouse (female, 6 - 7 weeks old). Nine days later, as the second stimulating agent, 10 μg/mouse of the compound of the invention was intravenously administered, and 90 minutes later, 0.5 to 1 mℓ of blood was sampled from the retro-orbital plexus. After being allowed to stand at room temperature for 5 to 6 hours, the blood was centrifuged at 7200 × g for 5 minutes to separate the serum. The serum was incubated at 56°C for 30 minutes for inactivation before use in the following experiment.

The TNF activity in serum was determined by measuring the damage of L929 cells were adjusted to a concentration of 6 × $10^4$ cells/well (0.1 mℓ) on the culture medium of RPMI 1640 containing 10% of fetal bovine serum (FBS) and 2 μg/mℓ of actinomycin D, and were seeded on a 96-well plates. The serum was diluted in steps in the RPMI 1640 medium with 10% FBS, and added to the cell by 0.1 mℓ for each. After incubation for 48 hours at 37°C, the viable cells were fixed with methanol. After staining the cells with 0.2% crystal violet, the cells were dissolved in 1% sodium dodecyl sulfate (SDS), and the absorbance at 550 nm was measured. The cytotoxicity (%) was calculated in the following formula, and the reciprocal of dilution factor of the serum showing 50% cytotoxicity was determined as the TNF in serum (U/mℓ).

$$\text{Cytotoxicity (\%)} = \frac{OD_{550}(\text{medium alone}) - OD_{550}(\text{with serum obtained by administering compounds of the invention})}{OD_{550}(\text{medium alone})} \times 100$$

The compound of the invention revealed activities shown in the following Table 2.

Table 2

| Compound | TNF titer in serum |
|---|---|
| Saline | < 10 |
| Control compound | 538900 |
| Example 1 | 727800 |

Experiment 3.

IL-1 producing activity

The producing activity of interleukin-1 (IL-1) was determined in the following experiment [see Biochem. Biopys. Res. Comm., Vol. 154, 1189 - 1196, 1988].

From 50 mℓ of human peripheral blood, buffy coat was isolated, and peripheral blood monocyte fraction was obtained by using Lymphoprep (Wako Pharmaceutical). After hemolyzing mixed erythrocytes, cells were rinsed twice in Dulbecco's modified Eagle medium (MEM), and suspended in the RPMI 1640 medium containing 5% fetal cow serum (FCS) and $2.5 \times 10^{-5}$ M 2-mercaptoethanol. The cells ($1 \times 10^6$ cells/well) were incubated in a 24-well plate containing 50 µg (50 µℓ) of the compound of the invention for 48 hours at 37°C in the presence of 5% carbon dioxide, and the supernatant of culture was samples as IL-1 induction supernatant. The IL-1 activity in the IL-1 induction supernatant was evaluated by measuring the growth inhibitory activity of IL-1 susceptible human melanoma cell A375 Met-Mix. The cells of A375 Met-Mix ($2 \times 10^3$ cells/0.1 mℓ/well) were suspended in RPMI 1640 medium containing 10% FCS, and the IL-1 induction supernatant (0.1 mℓ/well) diluted in steps was added and incubated in a 96-well plate for 96 hours at 37°C in the presence of 5% carbon dioxide. After incubation, the viable cells were fixed with methanol, and the cells were stained with 0.2% crystal violet, and the cells were dissolved with 1% SDS, and the absorbance at 550 nm was measured. The cell growth inhibitory rate (%) was determined in the following formula, and the reciprocal of the dilution factor of the IL-1 induction supernatant showing 50% inhibition was obtained as the IL-1 titer in serum (U/mℓ).

$$\text{Cell growth inhibitory rate (\%)} = \frac{OD_{550}\text{(medium alone)} - ODD_{500}\text{(with IL-1 induction supernatent)}}{ODD_{550}\text{(medium alone)}} \times 100$$

The compounds of the invention revealed activities shown in the following Table 3.

Table 3

| Compound | IL-1 titer U/mℓ |
|---|---|
| No compound | < 4 |
| Control compound | 15 |
| Example 1 | 24 |
| Example 4 | 16 |

Experiment 4.

Mitogen activity

The mitogen activity was evaluated in the following experiment [see Eur. J. Immunol., Vol. 14, 109 - 114, 1984].

The spleen of C3H/HeN mouse (male, 6 - 10 weeks old) was aseptically removed, rubbed and loosened in MEM, and passed through stainless steel mesh, and spleen cells were sampled. After hemolyzing the erythrocytes mixed in the spleen cells, the cells were suspended in RPMI 1640 medium containing 5% FBS and presented for the experiment.

The mitogen activity of the compound of the invention was evaluated by measuring the quantity of $^3$H-thymidine taken into the spleen cells treated with the compound of the invention. That is, the spleen cells adjusted to $5 \times 10^6$ cells/well (100 µℓ) were seeded in a 96-well plate, and the compound of the invention (100 µℓ) at specified concentration was added to the spleen cells. After incubation for 48 hours at 37°C in the presence of 5% carbon dioxide, 1 µCi/well (50 µℓ) of $^3$H-thymidine was added to the incubated cells, which were incubated for further 4 hours. The cells were washed in phosphate buffer saline (PBS), and the titer of $^3$H-thymidine (radioactivity) taken in the cells was determined by liquid scintillation counter. The

result was expressed in the stimulation index as follows.

$$\text{Stimulation index} = \frac{\text{Medium of compound of the invention (cpm)} - \text{Medium alone (cpm)}}{\text{Medium alone (cpm)}}$$

The compounds of the invention revealed activities shown in the following activities.

Table 4

| Compound | Simulation index | |
|---|---|---|
| | Compound concentration $\mu g/m\ell$ | |
| | 1 | 10 |
| No compound | 0 | 0 |
| Control compound | 2.7 | 18.3 |
| Example 1 | 14.8 | 26.5 |
| Example 4 | 5.1 | 19.6 |

Experiment 5.

Lethal toxicity by galactosamine loading

The lethal toxicity by galactosamine loading was evaluated in the following experiment [see J. Biochem., Vol. 98, 395 - 406, 1985].

To C57BL mouse (female, 7 weeks old), 10 mg of D-galactosamine hydrochloride was intraperitoneally administered, and the compound of the invention was administered in the caudal vein immediately after that. General conditions were observed in every hour for 7 hours after administration, and every day from the following day to the seventh day.

The compound of the invention showed lethal toxicity as following Table 5.

Table 5

| Compound | $LD_{50}$ (Galactosamine load) ($\mu g/kg$) |
|---|---|
| Control compound | 2.8 |
| Example 1 | 29.9 |

The preparation for the final objective compound [I] of the invention is practically described below while referring to some of the Examples thereof. But the invention is not limited to these Examples alone.

The relation between the compound numbers of the compound [I] of the invention and intermediate compounds [1] to [10] necessary for production thereof is as shown in the table below. Incidentally, the relation of 1 and m is interchangeable as evident from the structural formula, and for example, the relation of 1 = 11 and m = 13 is equal to that of 1 = 13 and m = 11 from other point of view.

Table 6

Compound [I] and its intermediate compounds [1] to [10], and compound numbers

| $R_1$ | l | m | n | p | $R_2$ | (1) | (2) | (3) | (4) | (5) | (8) | (9) | (10) | (6) | (7) | (I) | Example |
|---|---|---|---|---|---|---|---|---|---|---|---|---|---|---|---|---|---|
| H | 11 | 13 | 10 | 0 | $-CO(CH_2)_2CO_2CH_3$ | 1a | 2a | 3a | 4a | 5a | | | | 6a | 7a | A | 1 |
| H | 11 | 15 | 8 | 0 | $-CO(CH_2)_2CO_2CH_3$ | " | 2b | 3b | 4b | 5b | | | | 6b | 7b | B | 2 |
| H | 9 | 15 | 12 | 0 | $-CO(CH_2)_2CO_2CH_3$ | " | 2c | 3c | 4c | 5c | | | | 6c | 7c | C | 3 |
| H | 11 | 13 | 10 | 0 | $-COC(CH_3)_3$ | " | 2a | 3a | 4a | 5d | | | | 6d | 7d | D | 4 |
| H | 11 | 13 | 10 | 0 | $-CO(CH_2)_4CO_2CH_2CH_3$ | " | " | " | " | 5e | | | | 6e | 7e | E | 5 |
| H | 11 | 13 | 10 | 0 | $-COCH_3$ | " | " | " | " | 5f | | | | 6f | 7f | F | 6 |
| H | 11 | 13 | 10 | 0 | $-CO(CH_2)_2CO_2H$ | " | " | " | " | 5g | | | | 6g | 7g | G | 7 |
| H | 10 | 13 | 10 | 1 | $-COC(CH_3)_3$ | " | " | 3h | 4h | 5h | | | | 6h | 7h | H | 8 |
| H | 10 | 13 | 10 | 1 | $-COCH_3$ | " | " | " | " | 5i | | | | 6i | 7i | I | 9 |
| OH | 11 | 13 | 10 | 0 | $-COCH_3$ | | 2j | 3j | 4j | | 8j | 9j | 10j | 6j | 7j | J | 10 |
| OH | 11 | 13 | 10 | 0 | $-CO(CH_2)_2CO_2H$ | | " | " | " | | " | " | " | 6k | 7k | K | 11 |
| OH | 11 | 13 | 10 | 0 | $-CO(CH_2)_2CO_2CH_3$ | | " | " | " | 5l | | | | 6l | 7l | L | 12 |
| OH | 10 | 13 | 10 | 1 | $-COCH_3$ | | " | 3m | 4m | | 8m | 9m | 10m | 6m | 7m | M | 13 |
| OH | 10 | 13 | 10 | 1 | $-CO(CH_2)_2CO_2H$ | | " | " | " | | " | " | " | 6n | 7n | N | 14 |
| OH | 10 | 13 | 10 | 1 | $-CO(CH_2)_2CO_2CH_3$ | | " | " | " | 5o | | | | 6o | 7o | O | 15 |

Example 1.

1,5-Anhydro-6-O-{3-(methoxycarbonyl)propionyl}-2-deoxy-3-O-(2RS)-2-dodecylhexadecanoyl}-2-{(3R)-3-hydroxytetradecanamido}-4-O-phosphono-D-glucitol (compound A)

(First step)

1,5-Anhydro-2-deoxy-4,6-O-isopropyridene-2-[(3R)-3-{2-(trimethylsilyl)ethoxymethoxy}tetradecanamido]-D-glucitol (compound 2a)

5.8 g of 2-amino-1,5-anhydro-2-deoxy-4,6-O-isopropylidene-D-glucitol (1a), 10.7g of (R)-3-{2-(trimethylsilyl)ethoxymethoxy}tetradecanoic acid, and 11g of WSC•HCℓ are dissolved in 44 mℓ of dichloromethane, and the mixture was stirred under ice-cooling. The reaction was monitored by silica gel thin layer chromatography (chloroform : methanol = 20 : 1). After the reaction was completed, dichloromethane was added to the reaction mixture, which was washed with water and dried over anhydrous magnesium sulfate. The solvent was distilled off in vacuo, and the residue was purified by silica gel column chromatography (chloroform : methanol = 100 : 1) to yield a colorless crystalline compound (2a) (14g, yield 88%).

$[\alpha]_D$: -6.90° (c = 1.10, $CH_2C\ell_2$)

Melting point: 61.0 - 62.0°C

IR(nujol)$cm^{-1}$: 3450, 3280, 1640, 1550, 1460, 1380, 860 - 835

$^1$H-NMR(300MHz)δTMS $CDC\ell_3$: 0.03(9H, s, $Me_3Si$), 0.85 - 0.97(5H, m, $CH_2$TMS, -Me), 1.20 - 1.60(20H, m, $-CH_2-$), 1.43, 1.52(6H, each s, $-CMe_2$), 2.38, 2.48(2H, AB part of ABX, $J_{AB}$=14.9Hz, $J_{AX}$=6.6 Hz, $J_{BX}$=4.0Hz, $-CH_2CO-$), 3.22(2H, m, H-1, H-5), 3.44(1H, brs, -OH), 3.54 - 3.65(4H, m, H-1, H-4, $-C\underline{H_2}CH_2$TMS), 3.72(1H, t, J=10.5Hz, H-6), 3.87 - 3.92(2H, m, H-6, CH-OSEM), 4.01 - 4.09(2H, m, H-2, H-3), 4.67, 4.75(2H, AB, $J_{AB}$=6.6Hz, $-OCH_2O-$), 6.47(1H, d, J=7.0Hz, NH)

(Second step)

1,5-Anhydro-2-deoxy-3-O-{(2RS)-2-dodecylhexadecanoyl}-4,6-O-isopropylidene-2-[(3R)-3-{2-(trimethylsilyl)-ethoxymethoxy}tetradecanamido]-D-glucitol (compound 3a)

1.6g of compound (2a), 1.9g of (RS)-2-dodecylhexadecanoic acid, 173 mg of DMAP, and 1.1g of WSC•HCℓ are dissolved in 13.6 mℓ of dried dichloromethane, and the mixture was stirred for 12 hours at room temperature. The reaction mixture was added with dichloromethane, washed with water, dried over anhydrous magnesium sulfate, and concentrated in vacuo. The residue was purified by silica gel column chromatography (dichloromethane : methanol = 100 : 1 to 50 : 1) to yield an amorphous compound (3a) (2.5g, yield 90%).

IR(film)$cm^{-1}$: 3480, 2900, 1720, 1655, 1530, 1460, 1370

$^1$H-NMR(300MHz)δTMS $CDC\ell_3$: 0.03(9H, s, $Me_3Si$), 0.83 - 0.94(11H, m, $-CH_2$TMS, -Me), 1.12 - 1.75-(68H, m, $-CH_2-$), 1.32, 1.43(6H, each s, $CMe_2$), 2.15 - 2.40(3H, m, $-COCH_2-$, -COCH< ), 3.02 - 3.98(8H, m, $H_2$-1, H-4, H-5, $H_2$-6, $-OC\underline{H_2}CH_2$TMS), 4.15 - 4.22(2H, m, H-2, >CH-OSEM), 4.65(2H, s, $-O-CH_2-O-$), 4.92-(1H, t, J=9.6Hz, H-3), 6.18(1H, d, J=7.1Hz, NH)

(Third step)

1,5-Anhydro-2-deoxy-3-O-{(2RS)-2-dodecylhexadecanoyl-2-[(3R)-3-{2-(trimethylsilyl)-ethoxymethoxy}tetradecanamide]-D-glucitol (compound 4a)

1.9g of compound (3a) was dissolved in 95% aqueous acetic acid, and the mixture was stirred for 8 hours on 50°C warm bath. Toluene was added to the reaction mixture, which was concentrated in vacuo. The residue was purified by silica gel column chromatography (n-hexane : ethyl acetate = 2 : 1 - ethyl acetate 100%) to give an amorphous compound (4a) (1.2g, yield 64.8%).

IR(nujol)$cm^{-1}$: 4350, 4275, 1738, 1640, 1542

$^1$H-NMR(300MHz)δTMS $CDC\ell_3$: 0.02(9H, s, $Me_3Si$), 0.80 - 1.00(11H, m, $-CH_2$TMS, -Me), 1.10 - 1.71-(68H, m, $-CH_2-$), 2.12 - 2.50(3H, m, $-COCH_2-$, -COCH< ), 3.13(1H, t, J=10.3Hz, H-1), 3.26 - 3.37(1H, m, H-5), 3.51 - 3.99(6H, m, H-1, H-4, $H_2$-6, $-C\underline{H_2}CH_2$TMS), 4.01 - 4.20(2H, m, >CH-OSEM, H-2), 4.67(2H, s, -O-$CH_2$-O-), 4.84(1H, t, J=10.2Hz, H-3), 6.22(1H, d, J=7.3Hz, NH)

(Fourth step)

1,5-anhydro-6-O-{3-(methoxycarbonyl)propionyl-2-deoxy-3-O-{(2RS)-2-dodecylhexadecanoyl}-2-[(3R)-3-{2-(trimethylsilyl)ethoxymethoxy}tetradecanamido]-D-glucitol (compound 5a)

1.0g of compound (4a) was dissolved in 10 mℓ of dried pyridine. 0.14 mℓ of 3-(methoxycarbonyl)-propionyl chloride was added dropwise to the mixture with ice-cooling, and the mixture was stirred. After 30 minutes, the reaction mixture was returned to room temperature and was further stirred for 3 hours. The mixture was added with chloroform, then successively washed with water and aqueous NaCℓ, and dried over anhydrous magnesium sulfate. The solvent was distilled off in vacuo, and the residue was purified by silica gel column chromatography (n-hexane : ethyl acetate = 6 : 4) to give an amorphous compound (5a) (1.1g, yield 95.4%).

IR(film)$cm^{-1}$: 3356, 2928, 2858, 1738, 1657, 1543, 1460, 1249, 1158, 1104, 1031, 837, 665

$^1$H-NMR(300MHz)δTMS CDCℓ$_3$: 0.02(9H, s, $Me_3Si$), 0.80 - 1.00(11H, m, -$CH_2$TMS, -Me), 1.10 - 1.66-(68H, m, -$CH_2$-), 2.16 - 2.44(3H, m, -COCH< , -$COCH_2$-), 2.68(4H, s, -$COCH_2CH_2$CO-), 2.89(1H, d, J=4.8Hz, -OH), 3.09(1H, t,J=9.0Hz, H-1), 3.34 - 3.42(1H, m, H-5) 3.50 - 3.68(3H, m, -4, -$CH_2CH_2$TMS), 3.70(3H, s, -OMe), 3.86 - 3.92(1H, m, >CH-OSEM), 4.03 - 4.19(2H, m, H-2, H-1), 4.26, 4.55(2H, AB part of ABX, $J_{AB}$=12.2Hz, $J_{AX}$=3.7Hz, $J_{BX}$=1.7Hz), 4.85(1H, t, J=9.0Hz, H-3), 6.18(1H, d, J=7.5Hz, NH)

(Fifth step)

1,5-Anhydro-6-O-{3-(methoxycarbonyl)propionyl}-2-deoxy-4-O-diphenylphosphono-3-O-{(2RS)-2-dodecylhexadecanoyl-2-[(3R)-3-{2-(trimethylsilyl)ethoxymethoxy}tetradecanamido]-D-glucitol (compound 6a)

1.1g of compound (5a) and 264 mg of DMAP are dissolved in a mixed solvent of 1 mℓ of pyridine and 2 mℓ of dichloromethane. 0.54 mℓ of diphenyl phosphonochloride was added dropwise to the mixture under an argon atmosphere. After being stirred for an hour, the reaction mixture was returned to room temperature, and was further stirred for 5 hours. Chloroform was added to the reaction mixture, which was washed with water and concentrated in vacuo. The obtained residue was purified by silica gel column chromatography (n-hexane : ethyl acetate = 4 : 1) to afford amorphous compound (6a) (1.3g, yield 98%).

IR(film)$cm^{-1}$: 4300, 3858, 2858, 1744, 1686, 1541, 1460, 1249, 1164, 690, 665

$^1$H-NMR(300MHz)δTMS CDCℓ$_3$: 0.03(9H, s, -$Me_3Si$), 0.82 - 0.98(11H, m, -$CH_2$TMS, -Me), 1.00 - 1.65-(68H, m, -$CH_2$-), 2.15 - 2.38(3H, m, -$COCH_2$-, -COCH< ), 2.50 - 2.69(4H, m, -$COCH_2CH_2$CO-), 3.12(1H, t, J=12.4Hz, H-1), 3.50 - 3.71(6H, m, H-5, -$OCH_2CH_2$TMS, -OMe), 3.86 - 3.96(1H, m, >CH-OSEM), 4.02 - 4.10(1H, m, H-6), 4.11 - 4.22(2H, m, H-1, H-2), 4.27 - 4.40(1H, m, H-6), 4.67(2H, s, -O-$CH_2$-O-), 4.75(1H, q, J=9.5Hz, H-4), 5.18(1H, t, J=9.3Hz, H-3), 6.18(1H, d, J=7.1Hz, NH), 7.10 - 7.36(10H, m, Ph)

(Sixth step)

1,5-Anhydro-6-O-{3-(methoxycarbonyl)propionyl}-2-deoxy-4-O-diphenylphosphono-3-O-{(2RS)-2-dodecylhexadecanoyl}-2-{(3R)-3-hydroxytetradecanamido}-D-glucitol (compound 7a)

265 mg of compound 6a was dissolved in 27 mℓ of dried dichloromethane. 0.27 mℓ of boron trifluoride etherate was added to the mixture under an argon atmosphere with ice-cooling, and the mixture was stirred for 30 minutes. Dichloromethane was added to the mixture, which was washed successively with aqueous sodium bicarbonate and water, and dried over anhydrous magnesium sulfate. The solvent was distilled off in vacuo, and the obtained residue was purified by silica gel chromatography (n-hexane : ethyl acetate = 4 : 1) to afford an amorphous compound (7a) (153 mg, yield 64.3%).

IR(film)$cm^{-1}$: 3294, 2854, 1742, 1657, 1543, 1468, 1220, 1164, 690, 665

$^1$H-NMR(300MHz)δTMS CDCℓ$_3$: 0.88(9H, t, J=6.9Hz, -Me), 0.95 - 1.58(68H, m, -$CH_2$-), 2.11 - 2.40(3H, m, -$COCH_2$-, -COCH< ), 2.50 - 2.60(4H, m, -$COCH_2CH_2$CO-), 3.16(1H, t, J=13.7Hz, H-1), 3.26(1H, d, J=3.5Hz, -OH), 3.59 - 3.72(4H, m, H-5, -OMe), 3.86 - 3.98(1H, m, >CH-OH), 4.01 - 4.24(3H, m, H-1, H-2, H-6), 4.36(1H, d, J=10.6Hz, H-6), 4.76(1H, q, J=7.lHz, H-4), 5.18(1H, t, J=9.8Hz, H-3), 6.17(1H, d, J=7.1Hz, NH), 7.05 - 7.37(10H, m, Ph)

(Seventh step)

50 mg of compound (7a) was dissolved in a mixed solvent of methanol and ethanol (1:1, 20 mℓ). 40 mg of platinum oxide was added, and the mixture was stirred at room temperature for 12 hours under hydrogen atmosphere. After removal of the catalyst by filtration, the filtrate was concentrated in vacuo. The obtained residue was suspended in 1,4-dioxane, and lyophilyzed to give powder compound (A) (38 mg, yield 87.7%).

By $^1$H-NMR, the proton signal of the benzene ring was completely disappeared.

Melting point: 144.4 - 146.7 °C

IR(film) $cm^{-1}$: 2924, 2856, 1649, 1543, 1460, 1102

Example 2

1,5-Anhydro-6-O-{3-(methoxycarbonyl)propionyl}-2-deoxy-3-O-{(2RS)-2-dodecylocatadecanoyl}-2-{(3R)-3-hydroxydodecanamido}-4-O-phosphono-D-glucitol (compound B)

(First step)

1,5-Anhydro-2-deoxy-4,6-O-isopropylidene-2-[(3R)-3-{2-(trimethylsilyl)ethoxymethoxy}dodecanamido]-D-glucitol (compound 2b)

Using 1.1g of compound (1a) and 2.1g of (R)-3-{2-(trimethylsilyl)ethoxymethoxy}dodecanoic acid, and proceeding in the same way as compound (2a), a colorless crystalline compound (2b) was obtained (1.7g, yield 57.3%).

[α]D: -5.17° (c = 0.97, CHCℓ$_3$)

Melting point: 91 - 94 °C

IR: Same as in compound (2a)

$^1$H-NMR: Same as in compound (2a) except for -$CH_2$-integration.

(Second step)

1,5-Anhydro-2-deoxy-3-O-{(2RS)-2-dodecyloctadecanoyl]-4,6-O-isopropylidene-2-[(3R)-3-{2-(trimethylsilyl)-ethoxymethoxy}dodecanamido]-D-glucitol (compound 3b)

1g of compound (2b) and 853 mg of (2RS)-2-dodecyloctadecanoic acid and proceeding in the same way as compound (3a), an amorphous compound (3b) was obtained (1.22g, yield 67.1%).

IR: Same as in compound (3a).

$^1$H-NMR: Same as in compound (3a).

(Third step)

1,5-Anhydro-2-deoxy-3-O-{(2RS)-2-dodecyloctadecanoyl}-2-[(3R)-3-{2-(trimethylsilyl)-ethoxymethoxy}dodecanamido-D-glucitol (compound 4b)

Using 1.2g of compound (3b) and proceeding in the same way as compound (4a), an amorphous compound (4b) was obtained (838 mg, yield 71.7%).

IR: Same as in compound (4a).

$^1$H-NMR: Same as in compound (4a).

(Fourth step)

1,5-Anhydro-6-O-{3-(methoxycarbonyl)propionyl}-2-deoxy-3-O-(2RS)-2-dodecyloctadecanoyl}-2-[(3R)-3-{2-(trimethylsilyl)ethoxymethoxy}dodecanamido]-D-glucitol (compound 5b)

Using 1.0g of compound (4b) and proceeding in the same way as compound (5a), an amorphous compound (5b) was obtained (1.0g, yield 90.3%).

IR: Same as in compound (5a)

$^1$H-NMR: Same as in compound (5a)

(Fifth step)

1,5-Anhydro-6-O-{3-(methoxycarbonyl)propionyl}-2-deoxy-4-O-diphenylphosphono-3-O-{(2RS)-2-dodecyloctadecnaoyl}-2-[(3R)-3-{2-(trimethylsilyl)ethoxymethoxy}dodecanamido]-D-glucitol (compound 6b)

Using 1.0g of compound (5b) and proceeding in the same way as compound (6a), an amorphous compound (6b) was obtained (986 mg, yield 80.6%).
IR: Same as in compound (6a)
$^1$H-NMR: Same as in compound (6a)

(Sixth step)

1,5-Anhydro-6-O-{3-(methoxycarbonyl)propionyl}-2-deoxy-4-O-diphenylphosphono-3-O-{(2RS)-2-dodecyloctadecanoyl}-2-{(3R)-3-hydroxydodecanamido}-D-glucitol (compound 7b)

Using 986 mg of compound (6b) and proceeding in the same way as compound (7a), an amorphous compound (7b) was obtained (642 mg, yield 96.0%).
IR: Same as in compound (7a)
$^1$H-NMR: Same as in compound (7a)

(Seventh step)

Using 50 mg of compound (7b) and proceeding in the same way as compound (A), a white powder compound (B) was obtained (42 mg, yield 96.0%).
By $^1$H-NMR, the proton signal of the benzene ring was completely disappeared.
Melting point: 148.7 - 150.3 °C
IR: Same as in compound (A)

Example 3

1,5-Anhydro-6-O-{3-(methoxycarbonyl)propionyl}-3-O-{(2RS)-2-decyloctadecanoyl}-2-deoxy-2-{(3RS)-3-hydroxyhexadecanamido}-4-O-phosphono-D-glucitol (compound C)

(First step)

1,5-Anhydro-2-deoxy-4,6-O-isopropylidene-2-[(3RS)-3-{2-(trimethylsilyl)ethoxymethoxy}hexadecanamido]-D-glucitol (compound 2c)

Using 1.1g of compound (1a) and 2.5g of (RS)-3-{2-(trimethylsilyl)ethoxymethoxy)hexadecanoic acid and proceeding in the same way as compound (2a), an amorphous compound (2c) was obtained (2.6g, yield 81.7%).
IR: Same as in compound (2a).
$^1$H-NMR: Same as in compound (2a) except for integration value of $-CH_2-$.

(Second step)

1,5-Anhydro-3-O-{(2RS)-2-decyloctadecanoyl}-2-deoxy-4,6-O-isopropylidene-2-[(3RS)-3-{2-(trimethylsilyl)-ethoxymethoxy}hexadecanamido]-D-glucitol (compound 3c)

Using 1g of compound (2c) and 724 mg of (RS)-2-decyloctadecanoic acid and proceeding in the same way as compound (3a), an amorphous compound (3c) was obtained (822 mg, yield 48.6%) was obtained.
IR: Same as in compound (3a).
$^1$H-NMR: Same as in compound (3a) except for integration value of $-CH_2-$.

(Third step)

1,5-Anhydro-3-O-{(2RS)-2-decyloctadecanoyl}-2-deoxy-2-[(3RS)-3-{2-(trimethylsilyl)-ethoxymethoxy}hexadecanamido]-D-glucitol (compound 4c)

Using 822 mg of compound (3c) and proceeding in the same way as compound (4a), an amorphous compound (4c) was obtained (565 mg, yield 76.6%).

IR: Same as in compound (4a).

$^1$H-NMR: Same as in compound (4a) except for integration value of $-CH_2-$.

(Fourth step)

1,5-Anhydro-6-O-{3-(methoxycarbonyl)propionyl}-3-O-{(2RS)-2-decyloctadecanoyl}-2-deoxy-2-[(3RS)-3-{2-(trimethylsilyl)ethoxymethoxy}hexadecanamido]-D-glucitol (compound 5c)

Using 1g of compound (4c) and proceeding in the same way as compound (5a), an amorphous compound (5c) was obtained (918 mg, yield 83.2%).

IR: Same as in compound (5a).

$^1$H-NMR: Same as in compound (5a) except for integration value of $-CH_2-$.

(Fifth step)

1,5-Anhydro-6-O-{3-(methoxycarbonyl)propionyl}-3-O-{(2RS)-2-decyloctadecanoyl}-2-deoxy-4-O-diphenylphosphono-2-[(3RS)-3-{2-(trimethylsilyl)ethoxymethoxy}hexadecanamido]-D-glucitol (compound 6c)

Using 918 mg of compound (5c) and proceeding in the same way as compound (6a), an amorphous compound (6c) was obtained (1.1g, yield 94.7%).

IR: Same as in compound (6a)

$^1$H-NMR: Same as in compound (6a) except for integration value of $-CH_2-$.

(Sixth step)

1,5-Anhydro-6-O-{3-(methoxycarbonyl)propionyl}-3-O-{(2RS)-2-decyloctadecanoyl}-2-deoxy-4-O-diphenylphosphono-2-{(3RS)-3-hydroxyhexadecanamido}-D-glucitol (compound 7c)

Using 1.1g of compound (6c) and proceeding in the same way as compound (7a), an amorphous compound (7c) was obtained (570 mg, yield 57.6%).

IR: Same as in compound (7a)

$^1$H-NMR: Same as in compound (7a) except for integration value of $-CH_2-$.

(Seventh step)

Using 50 mg of compound (7c) and proceeding in the same way as compound (A), a white powder compound (C) was obtained (33 mg, yield 75.8%).

By $^1$H-NMR, the proton signal of the benzene ring was completely disappeared.

Melting point: 152.3 - 154.7 °C

IR: Same as in compound (A)

Example 4

1,5-Anhydro-2-deoxy-3-O-{(2RS)-2-dodecylhexadecanoyl}-2-{(3R)-3-hydroxytetradecanamido}-4-O-phosphono-6-O-pivaloyl-D-glucitol (compound D)

(Fourth step)

1,5-Anhydro-2-deoxy-3-O-{(2RS)-2-dodecylhexadecanoyl}-6-O-pivaloyl-2-[(3R)-3-{2-(trimethylsilyl)-ethoxymethoxy}tetradecanamido]-D-glucitol (compound 5d)

Using 300 mg of compound (4a) obtained in Third step of Example 1 and 0.04 mℓ of pivaloyl chloride, and proceeding in the same way as compound (5a), an amorphous compound (5d) was obtained (264 mg, yield 80.7%).

$^1$H-NMR(300MHz)δTMS $CDC\ell_3$: 0.02(9H, s, $Me_3Si$), 0.72 - 1.00(11H, m, $-CH_2TMS$, Me), 1.15 - 1.69-(66H, m, $-CH_2-$), 1.25(9H, s, $CMe_3$), 2.17 - 2.44(3H, m, -COCH< , $-COCH_2-$), 2.90(1H, d, -OH), 3.09(1H, t, J = 11.5Hz, H-1), 3.30 - 3.49(2H, m, H-4, H-5), 3.50 - 3.70(2H, m, $CH_2CH_2TMS$), 3.86 - 3.97(1H, m, >CHOSEM), 3.99 - 4.19(2H, m, H-1, H-2), 4.27, 4.52(2H, AB part of ABX, $J_{AB}$ = 12.7Hz, $J_{AX}$ = 1.4Hz, $J_{BX}$ = 2.8Hz, $H_2$-6), 4.66, 4.67(2H, each s, $-OCH_2O-$), 4.86(1H, t, J = 9.0Hz, H-3), 6.18(1H, d, J = 8.9Hz, NH)

(Fifth step)

1,5-Anhydro-2-deoxy-4-O-diphenylphosphono-3-O-{(2RS)-2-dodecylhexadecanoyl}-6-O-pivaloyl-2-[(3R)-3-{2-(trimethylsilyl)ethoxymethoxy}tetradecanamido]-D-glucitol(compound 6d)

Using 264 mg of compound (5d) and proceeding in the same way as compound (6a), an amorphous compound (6d) was obtained (310 mg, yield 97.8%).

$^1$H-NMR(300MHz)δTMS $CDC\ell_3$: 0.02(9H, s, $Me_3Si$), 0.82 - 0.97(11H, m, $-CH_2TMS$, Me), 1.15, (9H, s, $CMe_3$), 0.87 - 1.62(68H, m, $-CH_2-$), 2.16 - 2.40(3H, m, -COCH< , $-COCH_2-$), 3.11(1H, t, J = 12.1Hz, H-1), 3.50 - 3.72(3H, m, H-5, $CH_2CH_2TMS$), 3.86 - 4.22(4H, m, H-1, H-2, H-6, >CHOSEM), 4.28 - 4.41(1H, m, H-6), 4.67(2H, s, $-OCH_2O-$), 4.76(1H, q, J = 9.4Hz, H-4), 5.18(1H, t, J = 9.4Hz, H-3), 6.18(1H, d, J = 7.2Hz, NH), 7.04 - 7.39(10H, m, Ph)

(Sixth step)

1,5-Anhydro-2-deoxy-4-O-diphenylphosphono-3-O-{(2RS)-2-dodecylhexadecanoyl}-2-{(3R)-3-hydroxytetradecanamido}-D-glucitol (compound 7d)

Using 310 mg of compound (6d) and proceeding in the same way as compound (7a), an amorphous compound (7d) was obtained (213 mg, yield 76.8%).

IR(film)$cm^{-1}$: 3318, 2926, 1736, 1663, 1460, 1220, 1164, 690, 667

$^1$H-NMR(300MHz)δTMS $CDC\ell_3$: 0.88(9H, t, J = 6.2Hz, Me), 1.16(9H, s, $CMe_3$), 0.98 - 1,59(66H, m, $-CH_2-$), 2.11-2.42(3H, m, -COCH< , $-COCH_2-$), 3.16(1H, t, J = 12.2Hz, H-1), 3.27(1H, d, J = 3.6Hz, OH), 3.58 - 3.69(1H, m, H-5), 3.85 - 4.05(2H, m, H-6, -CHOH), 4.09 - 4.25(2H, m, H-1, H-2), 4.37(1H, d, J = 12.4Hz, H-6), 4.87(1H, q, J = 9.2Hz, H-4), 5.19(1H, t, J = 9.9Hz, H-3), 6.18(1H, d, J = 6.9Hz, NH), 7.08 - 7.40(10H, m, Ph)

(Seventh step)

Using 115 mg of compound (7d) and proceeding in the same way as compound (A), a white powder compound (D) was obtained (94 mg, yield 94.8%).

by $^1$H-NMR, the proton signal of benzene was completely disappeared.

Melting point: 104.3 - 107.3 °C

IR (film) $cm^{-1}$: 3288, 2926, 2856, 1719, 1649, 1560, 1543, 1460, 1296

Example 5

1,5-Anhydro-6-O-{5-(ethoxycarbonyl)pentanoyl}-2-deoxy-3-O-{(2RS)-2-dodecylhexadecanoyl}-2{(3R)-3-hydroxytetradecanamido}-4-O-phosphono-D-glucitol (compound E)

(Fourth step)

1,5-Anhydro-6-O-(5-(ethoxycarbonyl)pentanoyl}-2-deoxy-3-O-{(2RS)-2-dodecylhexadecanoyl}-2[(3RS)-3-{2-(trimethylsilyl)ethoxymethoxy}tetradecanamido]-D-glucitol (compound 5e)

500 mg of compound (4a) obtained in Third step of Example 1, 1.95 mg of ethyl adipate, 155 mg of WSC•HCℓ, and 33 mg of DMAP were dissolved in 5 mℓ of dichloromethane, the mixture was stirred for 12 hours at room temperature. The reaction mixture was directly chromatographed on silica gel (n-hexane : ethyl acetate = 2 : 1) to be purified, thereby yielding oily compound (5e) (557 mg, yield 95.3%).

$^1$H-NMR(300MHZ)δTMS CDCℓ$_3$: 0.02(9H, s, SiMe$_3$), 0.82 - 1.00(11H, m, -CH$_2$TMS, Me), 1.12 - 1.77-(72H, m, -CH$_2$-), 2.20 - 2.47(7H, m, -COCH< , -COCH$_2$-), 2.99(1H, d, J=4.8Hz, -OH), 3.11(1H, t, J=10.7Hz, H-1), 3.15 - 3.42(1H, m, H-5), 3.49 - 3.51(3H, m, H-4, -CH$_2$CH$_2$TMS), 3.86 - 3.98(1H, m, >CHOSEM), 4.04 - 4.21(4H, m, H-1, H-2, -COCH$_2$CH$_3$), 4.25 - 4.50(1H, m, H$_2$-6), 4.67(2H, s, -OCH$_2$O-), 4.85(1H, t, J=9.6Hz, H-3), 6.17(1H, d, J=7.3Hz, >NH)

(Fifth step)

1,5-Anhydro-6-O-{5-(ethoxycarbonyl)pentanoyl}-2-deoxy-4-O-diphenylphosphono-3-O-{(2RS)-2-dodecylhexadecanoyl}-2-[(3R)-3-{2-(trimethylsilyl)ethoxymethoxy}tetradecanamido]-D-glucitol (compound 6e)

Using 557 mg of compound (5e) and proceeding in the same way as compound (6a), an oily compound (6e) was obtained (448 mg, yield 66.2%).

$^1$H-NMR(300MHz)δTMS CDCℓ$_3$: 0.02(9H, s, SiMe$_3$), 1.80 - 1.00(11H, m, -CH$_2$TMS, Me), 2.18 - 2.40(7H, m, -COCH< , COCH$_2$-), 3.07 - 3.18(1H, m, H-1), 3.48 - 3.71(3H, m, H-5, -CH$_2$CH$_2$TMS), 3.86 - 3.96(1H, m, >CHOSEM), 4.02 - 4.39(6H, m, H-1, H-2, H$_2$-6, -COCH$_2$CH$_3$), 4.67(2H, s, -OCH$_2$O-), 4.75(1H, q, J=9.9Hz, H-4), 5.17(1H, t, J=9.7Hz, H-3), 6.17(1H, d, J=7.3Hz, >NH), 7.08 - 7.39(10H, m, Ph)

(Sixth step)

1,5-Anhydro-6-O-{5-(ethoxycarbonyl)pentanoyl}-2-deoxy-4-O-diphenylphosphono-3-O-{(2RS)-2-dodecylhexadecanoyl}-2-{(3R)-3-hydroxytetradecanamido}-D-glucitol (compound 7e)

Using 448 mg of compound (6e) and proceeding in the same way as compound (7a), compound (7e) was obtained (348 mg, yield 86.2%).

$^1$H-NMR(300MHz)δTMS CDCℓ$_3$: 0.88(9H, t, J=6.3Hz, Me) 0.98 - 1.72(72H, m, -CH$_2$-), 2.10 - 2.61(7H, m, -COCH< , -COCH$_2$-), 3.16(2H, t, J=12.2Hz, H-1), 3.26(1H, d, J=3.2Hz, -OH), 3.59 - 3.68(1H, m, H-5), 3.86 - 3.97(1H, m, >CHOH), 4.03 - 4.40(6H, m, H-1, H-2, H$_2$-6, -COCH$_2$CH$_3$), 4.77(1H, q, J=9.5Hz, H-4), 5.18(1H, t, J=9.8Hz, H-3), 6.33(1H, d, J=8.1Hz, >NH), 7.07 - 7.40(10H, m, Ph)

(Seventh step)

Using 50 mg of compound (7e) and proceeding in the same way as compound (A), a white powder compound (E) was obtained (30 mg, yield 68.8%).

By $^1$H-NMR, the proton signal of the benzene ring was completely disappeared.

Melting point: 83.2 - 84.6 °C

IR (film) cm$^{-1}$: 3286, 2924, 2852, 1733, 1648, 1544, 1467, 1377, 1182

Example 6

6-O-Acetyl-1,5-anhydro-2-deoxy-3-O-{(2RS)-2-dodecylhexadecanoyl}-2-{(3R)-3-hydroxytetradecanamido}-4-O-phosphono-D-glucitol (compound F)

(Fourth step)

6-O-Acetyl-1,5-anhydro-2-deoxy-3-O-{(2RS)-2-dodecylhexadecanoyl}-2-[(3R)-3-{2-(trimethylsilyl)-ethoxymethoxy}tetradecanamido]-D-glucitol (compound 5f)

300 mg of compound (4a) obtained in Third step of Example 1 was dissolved in a mixed solvent of 6 mℓ of dichloromethane and 1 mℓ of pyridine, 0.034 mℓ of acetic anhydride was added dropwise to the solution with cooling at -20 °C and then the mixture was stirred. Twelve hours later, the reaction mixture was diluted with chloroform, and washed with water. The organic layer was dried over anhydrous magnesium sulfate, and concentrated in vacuo. The residue was purified by silica gel column chromatography (n-hexane : ethyl acetate = 2 : 1) to afford an amorphous compound (5f) (276 mg, yield 89.5%).

$^1$H-NMR(300MHz)δTSM $CDCℓ_3$: 0.02(9H, s, Me), 0.82 - 0.99(11H, m, $-CH_2TMS$, Me), 1.14 - 1.65(68H, m, $-CH_2-$), 2.00 - 2.44(6H, m, -COCH$<$ , $-COCH_2-$, $-COCH_3$), 2.79(1H, d, J=4.5Hz, -OH), 3.11(1H, t, J=11.0Hz, H-1), 3.32 - 3.41(1H, m, H-5), 3.48 - 3.70(3H, m, H-4, $-CH_2CH_2TMS$), 3.84 - 3.96(1H, m, $>$CHOSEM), 4.03 - 4.19(2H, m, H-1, H-2), 4.38, 4.45(1H, AB part of ABX, $J_{AB}$=12.2Hz, $J_{AX}$=1.9Hz, $J_{BX}$=4.2Hz, $H_2$-6), 4.65, 4.68(2H, AB, $J_{AB}$=6.8Hz, $-OCH_2O-$), 4.85(1H, t, J=9.5Hz, H-3), 6.20(1H, d, J=7.2Hz, $>$NH)

(Fifth step)

6-O-Acetyl-1,5-anhydro-2-deoxy-4-O-diphenylphosphono-3-O-{(2RS)-2-dodecylhexadecanoyl}-2-[(3R)-3-{2-(trimethylsilyl)ethoxymethoxy}tetradecanamido]-D-glucitol (compound 6f)

Using 276 mg of compound (5f) and proceeding in the same way as compound (6a), compound (6f) was obtained (300mg, yield 87.4%).

$^1$H-NMR(300MHz)δTMS $CDCℓ_3$: 0.02(9H, s, SiMe), 0.81 - 0.98 (11H, m, $-CH_2TMS$, Me), 0.98 - 1.59-(68H, m, $-CH_2-$), 1.96(3H, s, $-COCH_3$), 2.18 - 2.39(4H, m, $-COCH_2-$), 3.06 - 3.20(1H, m, H-1), 3.50 - 3.69(3H, m, H-5, $-CH_2CH_2TMS$), 3.85 - 4.37(5H, m, H-1, H-2, $H_2$-6, $>$CHOSEM), 4.65(2H, s, $-OCH_2O-$), 4.75(1H, q, J=9.5Hz, H-4) , 5.19(1H, t, J=9.7Hz, H-3) , 6.27(1H, d, J=9.0Hz, $>$NH), 7.06 - 7.36(10H, m, Ph)

(Sixth step)

6-O-Acetyl-1,5-anhydro-2-deoxy-4-O-diphenylphosphono-3-O-{(2RS)-2-dodecylhexadecanoyl}-2-{(3R)-3-hydroxytetradecanamido}-D-glucitol (compound 7f)

Using 300 mg of compound (6f) and proceeding in the same way as compound (7a), compound (7f) was obtained (130 mg, yield 48.7%).

$^1$H-NMR(300MHz)δTMS $CDCℓ_3$: 0.88(9H, t, J=6.5Hz, Me), 0.96 - 1.64(68H, m, $-CH_2-$), 2.01(3H, s, $-COCH_3$), 2.13 - 2.41(3H, m, -COCH$<$ , $-COCH_2-$), 3.90 - 3.27(2H, m, H-1, -OH), 3.59 - 3.67(1H, m, H-5), 3.87 - 3.98(1H, m, $>$CHOH), 4.10 - 4.39(4H, m, H-1, H-2, $H_2$-6), 4.79(1H, q, J=9.4Hz, H-4), 5.18(1H, t, J=9.5Hz, H-3), 6.16(1H, d, J=7.1Hz, $>$NH), 7.09 - 7.47(10H, m, Ph)

(Seventh step)

Using 50 mg of compound (7f) and proceeding in the same way as compound (A), a white powder compound (F) was obtained (30 mg, yield 70.1%).

By $^1$H-NMR, the proton signal of the benzene ring was completely disappeared.

Melting point: 166.3 - 168.5 °C

IR (film) $cm^{-1}$: 3288, 2920, 2850, 1730, 1648, 1543, 1466, 1375, 1260, 1164

Example 7

1,5-Anhydro-6-O-(3-carboxypropionyl)-2-deoxy-3-O-{(2RS)-2-dodecylhexadecanoyl}-2-{(3R)-3-hydroxytetradecanamido}-4-O-phosphono-D-glucitol (compound G)

(Fourth step)

1,5-Anhydro-6-O-{3-(benzyloxycarbonyl)propionyl}-2-deoxy-3-O-{(2RS)-2-dodecylhexadecanoyl}-2-[(3R)-3-{2-(trimethylsilyl)ethoxymethoxy}tetradecanamido]-D-glucitol (compound 5g)

Using 800 mg of compound (4a) and 180 mg of 3-(benzyloxycarbonyl)propionic acid, and proceeding in the same way as compound (5e), an oily compound (5g) was obtained (860 mg, yield 96.4%).

$^1$H-NMR(300MHz)δTMS $CDC\ell_3$: 0.02(9H, s, -$SiMe_3$), 0.81 - 1.00(11H, m, -$CH_2$TMS, Me), 1.13 - 1.69-(68H, m, -$CH_2$-), 2.16 - 2.42(3H, m, -COCH< , -$COCH_2$-), 2.63 - 2.55(4H, m, -$COCH_2CH_2$CO-), 3.02 - 3.16-(1H, m, H-1), 3.31 - 3.71(4H, m, H-4, H-5, -$CH_2CH_2$TMS), 3.87 - 3.98(1H, m, >CHOSEM), 4.03 - 4.21(2H, m, H-1, H-2), 4.25 - 4.32(1H, m, H-6), 4.48 - 4.58(1H, m, H-6), 4.66(2H, s, -$OCH_2$O-), 4.84(1H, t, J = 9.7Hz, H-3), 5.09 - 5.19(2H, m, -$CH_2$Ph), 6.16(1H, d, J = 7.1Hz, >NH), 7.29 - 7.46(5H, m, Ph)

(Fifth step)

1,5-Anhydro-6-O-{3-(benzyloxycarbonyl)propionyl}-2-deoxy-4-O-diphenylphosphono-3-O-{(2RS)-2-dodecylhexadecanoyl)-2-{(3R)-3-{2-(trimethylsilyl)ethoxymethoxy}tetradecanamido]-D-glucitol (compound 6g)

Using 860 mg of compound (5g) and proceeding in the same way as compound (6a), an amorphous compound (6g) was obtained (987 mg, yield 95.0%).

$^1$H-NMR(300MHz)δTMS $CDC\ell_3$: 0.02(9H, s, -SiMe), 0.82 - 0.99(11H, m, -$CH_2$TMS, -Me), 0.99 - 1.41-(68H, m, -$CH_2$-), 2.15 - 2.39(3H, m, -COCH< , -$COCH_2$-), 2.51 - 2.73(4H, m, -$COCH_2CH_2$CO-), 3.10(1H, t, J = 12.5Hz, H-1), 3.49 - 3.71(3H, m, H-5, -$CH_2CH_2$TMS), 3.85 - 3.98(1H, m, >CHOSEM), 4.01 - 4.41(4H, m, H-1, H-2, $H_2$-6), 4.67(2H, s, -$OCH_2$O-), 4.74(1H, q, J = 9.6Hz, H-4), 5.11(2H, s, -$CH_2$Ph), 5.17(1H, t, J = 9.8Hz, H-3), 6.17(1H, d, J = 7.0Hz, >NH), 7.07 - 7.42(15H, m, Ph)

(Sixth step)

1,5-Anhydro-6-O-{3-(benzyloxycarbonyl)propionyl}-2-deoxy-4-O-diphenylphosphono-3-O-{(2RS)-2-dodecylhexadecanoyl}-2-{(3R)-3-hydroxytetradecanamido}-D-glucitol (compound 7g)

Using 987 mg of compound (6g) and proceeding in the same way as compound (7a), an amorphous compound (7g) was obtained (894 mg, yield 97.9%).

$^1$H-NMR(300MHz)δTMS $CDC\ell_3$: 0.88(9H, t, J = 6.5Hz, -Me), 0.98 - 1.52(68H, m, -$CH_2$-), 2.15 - 2.40(3H, m, -COCH< , -$COCH_2$-), 2.54 - 2.73(4H, m, -$COCH_2CH_2$CO-), 3.14(1H, t, J = 12.3Hz, H-1), 3.25(1H, d, J = 3.7Hz, -OH), 3.57 - 3.65(1H, m, H-5), 3.86 - 3.97(1H, m, >CHOH), 4.30 - 4.41(4H, m, H-1, H-2, $H_2$-6), 4.75(1H, q, J = 9.5Hz, H-4), 5.12(2H, s, -$CH_2$Ph), 5.17(1H, t, J = 9.8Hz, H-3), 6.13(1H, d, J = 6.3Hz, >NH), 7.08 - 7.42(15H, m, Ph)

1,5-Anhydro-6-O-(3-carboxypropionyl)-2-deoxy-4-O-diphenylphosphono-3-O-{(2RS)-2-dodecylhexadecanoyl}-2-{(3R)-3-hydroxytetradecanamido}-D-glucitol (compound 7g')

894 mg of compound (7g) was dissolved in 60 mℓ of ethanol, and 178 mg of 10% palladium carbon was added to the solution. The atmosphere in flask was replaced with hydrogen, and the mixture was stirred at room temperature. Thirty minutes later, the catalyst was filtered off, and the filtrate was concentrated in vacuo. The residue was purified by silica gel column chromatography (ethyl acetate 100% to ethyl acetate : methanol = 10 : 1) to give an amorphous compound (7g') (415 mg, yield 50.3%).

$^1$H-NMR(300MHz)δTMS $CDC\ell_3$: 0.88(9H, t, J = 6.2Hz, Me), 0.93 - 1.59(68H, m, -$CH_2$-), 2.19(2H, d, J = 6.1Hz, -$NHCOCH_2$-), 2.33(1H, t, J = 6.5Hz, -COCH< ), 2.42 - 2.85(4H, m, -$COCH_2CH_2$CO-), 3.16(1H, t, J = 11.2Hz, H-1), 3.59(1H, d, J = 9.5Hz, H-5), 3.77 - 4.08(5H, m, H-1, H-2, $H_2$-6, >CHOH), 4.83(1H, q, J = 9.5Hz, H-4), 5.27(1H, t, J = 9.7Hz, H-3), 7.01 - 7.41(11H, m, >NH, Ph)

(Seventh step)

Using 50 mg of compound (7g') and proceeding in the same way as compound (A), a white powder compound (G) was obtained (30 mg, yield 69.1%).

By $^1$H-NMR, the proton signal of the benzene ring was completely disappeared.

Melting point: 181.1 - 182.8 °C

IR (film) $cm^{-1}$: 3488, 3344, 2920, 2850, 2338, 1728 1666, 1547, 1467, 1164, 1041

Example 8

1,5-Anhydro-2-deoxy-2-{(3R)-3-hydroxytetradecanamido}-4-O-phosphono-6-O-pivaloyl-3-O-{(3RS)-3-undecylheptadecanoyl}-D-glucitol (compound H)

(Second step)

1,5-Anhydro-2-deoxy-4,6-O-isopropylidene-2-[(3R)-3-{2-(trimethylsilyl)ethoxymethoxy}tetradecanamido]-3-O-{(3RS)-3-undecylheptadecanoyl}-D-glucitol (compound 3h)

Using 3.0g of compound (2a) and 2.0g of (RS)-3-undecylheptadecanoic acid and proceeding in the same way as compound (3a), an amorphous compound (3h) was obtained (4.12g, yield 91.0%).

IR(film)$cm^{-1}$: 3320, 2900, 1735, 1645, 1545, 1470, 1383

$^1$H-NMR(300MHz)δTMS $CDCl_3$: 0.03(9H, s, $Me_3Si$), 0.86 - 0.97(11H, m, $-CH_2TMS$, -Me), 1.18 - 1.60-(66H, m, $-CH_2$-), 1.36, 1.47(6H, each s, $CMe_2$), 1.75 - 1.85(1H, m, -CH< ), 2.20 - 2.40(4H, m, $-CH_2CO$-), 3.10 - 4.00(8H, m, $H_2$-1, H-4, H-5, $H_2$-6, $-O-CH_2CH_2TMS$), 4.16(2H, m, H-2, >CHOSEM), 4.66, 4.68(2H, AB, $J_{AB}$ = 6.9Hz, $-OCH_2O$-), 4.94(1H, t, J = 9.6Hz, H-3), 6.27(1H, d, J = 7.0Hz, NH)

(Third step)

1,5-Anhydro-2-deoxy-2-[(3R)-3-{2-(trimethylsilyl)ethoxymethoxy}tetradecanamido]-3-O-{(3RS)-3-undecylheptadecanoyl}-D-glucitol (compound 4g)

Using 2.7g of compound (3h) and proceeding in the same way as compound (4a), an amorphous compound (4h) was obtained (1.84 g, yield 91.0%).

IR (film) $cm^{-1}$: 3600 - 3100, 2900, 1720, 1650, 1540, 1463, 1380

$^1$H-NMR(300MHz)δTMS $CDCl_3$: 0.02(9H, s, $-SiMe_3$), 0.85 - 0.96(11H, m, $-CH_2TMS$, -Me), 1.10 - 1.65-(66H, m, $-CH_2$-), 1.75 - 1.85(1H, m, -CH< ), 2.20 - 2.40(4H, m, $-COCH_2$-), 2.65(1H, brs, -OH), 3.13(1H, t, J = 10.0Hz, H-1), 3.32(1H, m, H-5), 3.52 - 3.95(6H, m, H-1, $-CH_2CH_2TMS$, H-4, $H_2$-6), 4.0 - 4.17(2H, m, H-2, >CHOSEM), 4.63, 4.69(2H, AB, J = 7.0Hz, $-OCH_2O$-), 4.85(1H, t, J = 9.4Hz, H-3), 6.29(1H, d, J = 7.3Hz, NH)

(Fourth step)

1,5-Anhydro-2-deoxy-6-O-pivaloyl-2-[(3R)-3-{2-(trimethylsilyl)ethoxymethoxy}tetradecanamido]-3-O-{(3RS)-3-undecylheptadecanoyl}-D-glucitol (compound 5h)

Using 400 mg of compound (4h) and proceeding in the same way as compound (5d), compound (5h) was obtained (405 mg, yield 92.8%).

$^1$H-NMR(300MHz)δTMS $CDCl_3$: 0.02(9H, s, SiMe), 0.88 - 0.98(11H, m, $-CH_2TMS$, Me), 1.12 - 1.91(76H, m, $-CH_2$-, t-bu, >CH-), 2.21 - 2.42(4H, m, $-COCH_2$-), 2.95(1H, brs, OH), 3.08(1H, t, J = 10.7Hz, H-1), 3.28 - 3.70(4H, m, H-4, H-5, $-CH_2CH_2TMS$), 3.82 - 3.93(1H, m, >CHOSEM), 3.96 - 4.19(2H, m, H-1, H-2), 4.19 - 4.28(1H, m, H-6), 4.48 - 4.57(1H, m, H-6), 4.64, 4.68(2H, AB, $J_{AB}$ = 6.8Hz, $-OCH_2O$-), 4.80 - 4.90(1H, m, H-3), 6.22(1H, d, J = 10.0Hz, >NH)

(Fifth step)

1,5-Anhydro-2-deoxy-4-O-diphenylphosphono-6-O-pivaloyl-2-[(3R)-3-{2-(trimethylsilyl)-ethoxymethoxy}tetradecanamido]-3-O-{(3RS)-3-undecylheptadecanoyl}-D-glucitol (compound 6h)

Using 405 mg of compound (5h) and proceeding in the same way as compound (6a), an amorphous compound (6h) was obtained (307 mg, yield 61.7%).

$^1$H-NMR(300MHz)δTMS $CDC\ell_3$: 0.02(9H, s, $-SiMe_3$), 0.83 - 1.00(11H, m, $-CH_2$TMS, -Me), 1.00 - 1.77-(76H, m, $-CH_2$-, t-Bu, >CH), 2.10 - 2.35(4H, m, $-COCH_2$-), 3.11(1H, t, J = 9.9Hz, H-1), 3.57 - 4.23(7H, m, H-1, H-2, H-5, H-6, >CHOSEM, $-CH_2CH_2$TMS), 4.30 - 4.39(1H, m, H-6), 4.66(2H, s, $-OCH_2O$-), 4.37(1H, q, J = 9.2Hz, H-4), 5.14(1H, t, J = 9.9Hz, H-3), 6.20(1H, d, J = 6.9Hz, >NH)

(Sixth step)

1,5-Anhydro-2-deoxy-4-O-diphenylphosphono-2-{(3R)-3-hydroxytetradecanamido}-6-O-pivaloyl-3-O-{(3RS)-3-undecylheptadecanoyl}-D-glucitol (compound 7h)

Using 307 mg of compound (6h) and proceeding in the same way as compound (7a), an amorphous compound (7h) was obtained (227 mg, yield 82.6%).

$^1$H-NMR(300MHz)δTMS $CDC\ell_3$: 0.86(9H, t, J = 6.2Hz, Me), 0.98 - 1.74(76H, m, $-CH_2$-, t-Bu, >CH-), 2.08 - 2.30(4H, m, $-COCH_2$-), 3.09 - 3.24(2H, m, H-1, OH), 3.52 - 3.61(1H, m, H-5), 3.85 - 4.22(4H, m, H-1, H-2, H-6, >CHOH), 4.28 - 4.37(1H, m, H-6), 4.73(1H, q, J = 9.3Hz, H-4), 5.07 - 5.18(1H, m, H-3), 6.17(1H, d, J = 7.2Hz, >NH), 7.08 - 7.35(10H, m, Ph)

(Seventh step)

Using 100 mg of compound (7h) and proceeding in the same way as compound (A), a white powder compound (H) was obtained (64 mg, yield 74.2%).

By $^1$H-NMR, the proton signal of the benzene ring was completely disappeared.

Melting point: 113.1 - 114.8 °C

IR (film) $cm^{-1}$: 3696, 2920, 2852, 1720, 1650, 1540, 1467, 1370, 1293, 1169, 1103, 1039

Example 9

6-O-Acetyl-1,5-anhydro-2-deoxy-2-{(3R)-3-hydroxytetradecanamido}-4-O-phosphono-3-O-{(3RS)-3-undecylheptadecanoyl}-D-glucitol (compound I)

(Fourth step)

6-O-Acetyl-1,5-anhydro-2-deoxy-2-[(3R)-3-{2-(trimethylsilyl)ethoxymethoxy}tetradecanamido]-3-O-{(3RS)-3-undecylheptadecanoyl}-D-glucitol (compound 5i)

Using 171 mg of compound (4h) obtained in Third step of Example 8 and proceeding in the same way as compound (5f), an oily compound (5i) was obtained (137 mg, yield 76.6%).

$^1$H-NMR(300MHz)δTMS $CDC\ell_3$: 0.02(9H, s, SiMe), 0.80 - 0.98(11H, m, $-CH_2$TMS, Me), 1.13 - 1.59(67H, m, $-CH_2$-, >CH-), 2.12(3H, s, $-COCH_3$), 2.21 - 2.40(4H, m, $-COCH_2$-), 2.98 - 3.17(2H, m, H-1, OH), 3.35 - 3.43(1H, m, H-5), 3.46 - 3.69(3H, m, H-4, $-CH_2CH_2$TMS), 3.82 - 3.93(1H, m, >CHOSEM), 4.04 - 4.18(2H, m, H-1, H-2), 4.32, 4.43(1H, AB part of ABX, $J_{AB}$ = 12.2Hz, $J_{AX}$ = 2.8Hz, $J_{BX}$ = 4.6Hz, $H_2$-6), 4.65, 4.68(2H, AB, $J_{AB}$ = 6.2Hz, $-OCH_2O$-), 4.82 - 4.92(1H, m, H-3), 6.29(1H, d, J = 9.0Hz, >NH)

(Fifth step)

6-O-Acetyl-1,5-anhydro-2-deoxy-4-O-diphenylphosphono-2-[(3R)-3-{2-(trimethylsilyl)-ethoxymethoxy}tetradecanamido]-3-O-{(3RS)-3-undecylheptadecanoyl}-D-glucitol (compound 6i)

Using 253 mg of compound (5i) and proceeding in the same way as compound (6a), an amorphous compound (6i) was obtained (258 mg, yield 32.3%).

$^1$H-NMR(300MHz)δTMS $CDC\ell_3$: 0.02(9H, s, -SiMe), 0.81 - 1.00(11H, m, $-CH_2$TMS, -Me), 1.00 - 1.75-

(67H, m, $-CH_2-$, >CH-), 2.00(3H, s, $-COCH_3$), 2.03 - 2.36(4H, m, $-COCH_2-$), 3.07 - 3.19(1H, m, H-1), 3.50 - 3.70(3H, m, H-5, $-CH_2CH_2TMS$), 3.84 - 3.95(1H, m, >CHOSEM), 3.98 - 4.35(4H, m, H-1, H-2, $H_2$-6), 4.64, 4.67(2H, AB, $J_{AB}$ = 6.8Hz, $-OCH_2O-$), 4.73(1H, q, J = 9.3Hz, H-4) 5.17(1H, t, J = 9.3Hz, H-3), 6.28(1H, d, J = 9.0Hz, >NH), 7.10 - 7.37(10H, m, Ph)

(Sixth step)

6-O-Acetyl-1,5-anhydro-2-deoxy-4-O-diphenylphosphono-2-{(3R)-3-hydroxytetradecanamido}-3-O-{(3RS)-3-undecylheptadecanoyl}-D-glucitol (compound 7i)

Using 258 mg of compound (6i) and proceeding in the same way as compound (7a), an amorphous compound (7i) was obtained (167 mg, yield 72.6%).

$^1$H-NMR(300MHz)δTMS $CDC\ell_3$: 0.88(9H, t, J = 6.2Hz, Me), 1.00 - 1.80(67H, m, $-CH_2-$, >CH-), 2.00 - 2.31(7H, m, $-COCH_2-$, $COCH_3$), 3.12 - 3.23(1H, m, H-1), 3.57 - 3.66(1H, m, H-5), 3.88 - 4.36(5H, m, H-1, H-2, $H_2$-6, >CHOH), 4.75(1, q, J = 9.3Hz, H-4), 5.16(1H, t, J = 9.2Hz, H-3), 6.20(1H, d, J = 9.0Hz, >NH), 7.10 - 7.48(10H, m, Ph)

(Seventh step)

Using 100 mg of compound (7i) and proceeding in the same way as compound (A), a white powder compound (I) was obtained (62 mg, yield 72.3%).

By $^1$H-NMR, the proton signal of the benzene ring was completely disappeared.

Melting point: 163.8 - 165.3 °C

IR (film) $cm^{-1}$: 3298, 2918, 2850, 1722, 1649, 1544, 1467, 1376, 1263, 1164, 1103, 1047

Example 10

6-O-Acetyl-2-deoxy-3-O-{(2RS)-2-dodecylhexadecanoyl}-2-{(3R)-3-hydroxytetradecanamido}-4-O-phosphono-D-glucopyranose (compound J)

(Second step)

2-(Trimethylsilyl)ethyl 2-deoxy-3-O-{(2RS)-2-dodecylhexadecanoyl}-4,6-O-isopropylidene-2-[(3R)-3-{2-(trimethylsilyl)ethoxymethoxy}tetradecanamido]-β-D-glucopyranoside (compound 3j)

Using 1.03g of known compound 2-(trimethylsilyl)ethyl 2-deoxy-4,6-O-isopropylidene-2-[(3R)-3-{2-(trimethylsilyl)ethoxymethoxy}tetradecanamido-β-D-glucopyranoside (2j) (Japanese patent Disclosure 62888/90) and 971 mg of (2RS)-2-dodecylhexadecanoic acid, and proceeding in the same way as compound (3a), a syrupy compound (3j) was obtained (1.33g, yield 80.6%).

IR(Film)$cm^{-1}$: 3300, 2930, 2850, 1740, 1650, 1550, 860, 840

$^1$H-NMR(300MHz)δTMS $CDC\ell_3$: 0.00(18H, m, MeSi), 0.86(13H, m, $CH_2TMS$, -Me), 1.25(68H, m, $-CH_2-$), 1.34, 1.45(6H, 2s, $-CMe_2$), 2.29(3H, m, $-COCH_2-$, -COCH< ), 3.40 - 4.02(8H, m, $CH_2CH_2TMS$, >CHOSEM, H-2, H-4, H-5, $H_2$-6), 4.55(1H, d, J = 8.4Hz, H-1), 4.64 - 4.75(2H, 2d, J = 13.5Hz, $-OCH_2O-$), 5.20(1H, t, J = 9.6Hz, H-3), 6.35(1H, d, J = 9.1Hz, >NH)

(Third step)

2-(Trimethylsilyl)ethyl 2-deoxy-3-O-{(2RS)-2-dodecylhexadecanoyl}-2-[(3R)-3-{2-(trimethylsilyl)-ethoxymethoxy}tetradecanamido]-β-D-glucopyranoside (compound 4j)

Using 1.26g of compound (3j) and proceeding in the same way as compound (4a), a syrupy compound (4j) was obtained (0.95g, yield 78.5%).

IR(film)$cm^{-1}$: 3300, 2940, 2860, 1740, 1650, 1550, 860, 840

$^1$H-NMR(300MHz)δTMS $CDC\ell_3$: 0.00(18H, m, MeSi), 0.84(13H, m, $CH_2TMS$, -Me), 1.24(68H, m, $-CH_2-$), 2.30(3H, m, $-COCH_2-$, -COCH-), 3.00(1H, d, J = 5.3Hz, OH), 3.42 - 3.95(8H, m, $CH_2CH_2TMS$, >CHOSEM, H-2, H-4, H-5, $H_2$-6), 4.64 - 4.72(3H, m, $-OCH_2O-$, H-1), 5.20(1H, t, J = 9.2Hz, H-3), 6.35(1H, d, J = 8.6Hz, >NH)

(Eighth step)

2-(Trimethylsilyl)ethyl 6-O-(tert)-butyldimethylsilyl-2-deoxy-3-O-{(2RS)-2-dodecylhexadecanoyl}-2-[(3R)-3-{2-(trimethylsilyl)ethoxymethoxy}tetradecanamido]-β-D-glucopyranoside (compound 8j)

550 mg of compound (4j) was dissolved in 15 mℓ of pyridine, 159 mg of TBSMS-Cℓ was added to the solution, and the mixture was stirred for 12 hours at room temperature. After methanol was added to the reaction mixture and the solvent was distilled off, and the obtained residue was purified by silica gel column chromatography (dichloromethane : methanol = 450 : 1) to give a syrupy compound (8j) (590 mg, yield 97.6%).

IR(Film)$cm^{-1}$: 3500, 3300, 2940, 2860, 1720, 1650, 1550, 860, 840

$^1$H-NMR(300MHz)δTMS CDCℓ$_3$: 0.00(24H, m, MeSi), 0.90(22H, m, $CH_2$TMS, -Me), 1.25(68H, m, $-CH_2-$), 2.27 - 2.38(3H, m, $-COCH_2-$, -COCH-), 3.15(1H, d, J=2.9Hz, OH), 3.39 - 3.95(8H, m, $CH_2CH_2$TMS, >CHOSEM, H-2, H-4, H-5, $H_2$-6), 4.56(1H, d, J=8.4Hz, H-1), 4.65 - 4.73(2H, d, J=9.5Hz, $-OCH_2O-$), 5.11-(1H, t, J=9.0Hz, H-3), 6.12(1H, d, J=9.0Hz, >NH)

(Ninth step)

2-(Trimethylsilyl)ethyl 6-O-(tert)-butyldimethylsilyl-2-deoxy-4-O-diphenylphosphono-3-O-{(2RS)-2-dodecylhexadecanoyl}-2-[(3R)-3-{2-(trimethylsilyl)ethoxymethoxy}tetradecanamido]-β-D-glucopyranoside (compound 9j)

540 mg of compound (8j) was dissolved in 6 mℓ of pyridine, 115 mg of DMAP and 252 mg of diphenylphosphonochloride were added to the solution, and the mixture was stirred for 12 hours at 40°C. After methanol was added to the reaction mixture and the solvent was distilled off, the residue was extracted with chloroform, then successively washed with 2N hydrochloric acid and water. The organic layer was dried over Gluaber's salt, and concentrated in vacuo. The obtained residue was purified by silica gel column chromatography (dichloromethane : methanol = 300 : 1) to give an amorphous compound (9j) (590 mg, yield 90.9%).

IR(Film)$cm^{-1}$: 3350, 2960, 2900, 1750, 1680, 1550, 960, 790-650

$^1$H-NMR(300MHz)δTMS CDCℓ$_3$: 0.00(24H,m, MeSi), 0.84(22H, m, $CH_2$TMS, -Me), 1.26(68H, m, $-CH_2-$), 2.29(3H, m, $-COCH_2-$, -COCH< ), 3.48 - 3.98(8H, m, $CH_2CH_2$TMS, >CHOSEM, H-2, H-4, H-5, $H_2$-6), 4.59 - 4.74(3H, m, $-OCH_2O-$, H-1), 5.52(1H, t, J=9.0Hz, H=3), 6.13(1H, d, J=8.8Hz, >NH), 7.12 - 7.35(10H, m, Ph)

(Tenth step)

2-(Trimethylsilyl)ethyl 2-deoxy-4-O-diphenylphosphono-3-O-{(2RS)-2-dodecylhexadecanoyl}-2-[(3R)-3-{2-(trimethylsilyl)ethoxymethoxy}tetradecanamido]-β-D-glucopyranoside (compound 10j)

550 mg of compound (9j) was dissolved in 30 mℓ of 80% acetic acid, and the mixture was stirred at 50°C. The reaction mixture was concentrated in vacuo, and the residue was purified by silica gel column chromatography (dichloromethane : methanol = 300 : 1) to give an amorphous compound (10j) (320 mg, yield 63.5%).

IR(Film)$cm^{-1}$: 3500, 3300, 2940, 2860, 1740, 1650, 1550, 960, 860, 840, 780-650

$^1$H-NMR(300MHz)δTMS CDCℓ$_3$: 0.00(18H, m, MeSi), 0.88(13H, m, $CH_2$TMS, -Me), 1.26(68H, m, $-CH_2-$), 2.30(3H, m, $-COCH_2-$, -COCH< ), 3.34 - 3.97(8H, m, $CH_2CH_2$TMS, >CHOSEM, H-2, H-4, H-5 $H_2$-6), 4.65 - 4.76(3H, m, $-OCH_2O-$, H-1), 5.56(1H, t, J=9.3Hz, H-3), 6.22(1H, d, J=8.4Hz, >NH), 7.13 - 7.36(10H, m, Ph)

(Eleventh step)

2-(Trimethylsilyl)ethyl 6-O-acetyl-2-deoxy-4-O-diphenylphosphono-3-O-{(2RS)-2-dodecylhexadecanoyl}-2-[-(3R)-3-{2-(trimethylsilyl)ethoxymethoxy}-tetradecanamido]-β-D-glucopyranoside (compound 6j)

140 mg of compound (10j) was dissolved in 2 mℓ of pyridine. After 13 mg of acetyl chloride was added to the solution with ice cooling, the mixture was stirred for 12 hours at room temperature. An excess reagent was decomposed by addition of methanol, and the solvent was distilled off. The obtained residue

was purified by silica gel column chromatography (dichloromethane : methanol = 400 : 1) to give an amorphous compound (6j) (140 mg, quant).

IR(Film)$cm^{-1}$: 3300, 2940, 2860, 1750, 1650, 1550, 950, 860, 840, 760-650

$^1$H-NMR(300MHz)δTMS $CDC\ell_3$: 0.00(18H, m, MeSi), 0.88(13H, m, $CH_2$TMS, -Me), 1.25(68H, m, -$CH_2$-), 1.97(3H, s, -$COCH_3$), 2.30(3H, m, -$COCH_2$-, -COCH< ), 3.49 - 3.90(6H, m, $CH_2CH_2$TMS, >CHOSEM, H-2, H-4, H-5), 4.12(1H, dd, J=12.2Hz, J=4.3Hz, H-6), 4.34(1H, d, J=10.2Hz, H-6), 4.65 - 4.82(3H, m, -$OCH_2O$-, H-1), 5.59(1H, t, J=9.1Hz, H-3), 6.21(1H, d, J=8.4Hz, >NH), 7.09 - 7.34(10H, m, Ph)

(Sixth step)

6-O-Acetyl-2-deoxy-4-O-diphenylphosphono-3-O-{(2RS)-2-dodecylhexadecanoyl}-2-{(3R)-3-hydroxytetradecanamido-D-glucopyranose (compound 7j)

Using 140 mg of compound (6j) and proceeding in the same way as compound (7a), an amorphous compound (7j) was obtained (75.7 mg, yield 65.7%).

Melting point: 80 - 82 °C

IR(Film)$cm^{-1}$: 3300, 2940, 2860, 1750, 1650, 1540, 960

$^1$H-NMR(300MHz)δTMS $CDC\ell_3$: 0.88(9H, m, -Me), 1.25(68H, m, -$CH_2$-), 1.99(3H, s, -$COCH_3$), 2.14 - 2.34(3H, m, -$COCH_2$-, COCH< ), 3.64(1H, br, OH), 3.97 - 4.32(5H, m, >CHOSEM, H-2, H-5, $H_2$-6), 4.68-(1H, q, J=9.5Hz, H-4), 5.21(1H, br, H-1), 5.52(1H, t, J=9.1Hz, H-3), 6.35(1H, d, J=8.8Hz, >NH), 7.10 - 7.33(10H, m, Ph)

(Seventh step)

Using 75.3 mg of compound (7j) and proceeding in the same as compound (A), compound (J) was obtained (66.7 mg, quant).

Melting point: 170 - 172 °C

IR (KBr) $cm^{-1}$: 3300, 2940, 2860, 1740, 1640, 1550

Example 11

2-Deoxy-3-O-{(2RS)-2-dodecylhexadecanoyl}-6-O-(3-carboxypropionyl)-2-{(3R)-3-hydroxytetradecanamido}-4-O-phosphono-D-glucopyranose (compound K)

(Eleventh step)

2-(Trimethylsilyl)ethyl 2-deoxy-4-O-diphenylphosphono-3-O-{(2RS)-2-dodecylhexadecanoyl}-6-O-(3-carboxypropionyl)-2-[(3R)-3-{2-(trimethylsilyl)ethoxymethoxy}tetradecanamido]-β-D-glucopyranoside (compound 6k)

130 mg of compound (10j) obtained in Tenth step of Example 10 was dissolved in 3 mℓ of 1,2-dichloroethane. 30 mg of succinic anhydride and catalytic amount of DMAP were added to the solution, and the reaction mixture was stirred for 3 hours at room temperature. The mixture was concentrated in vacuo, and the obtained residue was purified by silica gel column chromatography (dichloromethane : methanol = 50 : 1) to give an amorphous compound (6k) (125.5 mg, yield 89.4%).

IR(Film)$cm^{-1}$: 3300, 2930, 2850, 1740, 1660, 1550, 950, 860, 840, 760, 650

$^1$H-NMR(300MHz)δTMS $CDC\ell_3$: 0.00(18H, m, MeSi), 0.88(13H, m, $CH_2$TMS, -Me), 1.25(68H, m, -$CH_2$-), 2.27 - 2.64(7H, m, -$COCH_2$-, -COCH< ), 3.52 - 3.93(6H, m, -$CH_2CH_2$TMS, >CHOSEM, H-2, H-4, H-5), 4.09(1H, dd, J=12.1Hz, J=3.9Hz, H-6), 4.39(1H, d, J=10.4Hz, H-6), 4.65 - 4.85(3H, m, -$OCH_2O$, H-1), 5.56-(1H, t, J=9.5Hz, H-3), 6.28(1H, d, J=8.8Hz, >NH), 7.11 - 7.32(10H, m, Ph)

(Sixth step)

2-Deoxy-4-O-diphenylphosphono-3-O-{(2RS)-2-dodecylhexadecanoyl}-6-O-(3-carboxypropionyl)-2-{(3R)-3-hydroxytetradecanamido}-D-glucopyranose (compound 7k)

Using 116.8 mg of compound (6k) and proceeding in the same way as compound (7a), an amorphous compound (7k) was obtained (86.2 mg, yield 88.8%).

Melting point: 71 - 73 °C

IR(Film)cm$^{-1}$: 3300, 2930, 2850, 1740, 1650, 1550, 960, 770 - 650

$^1$H-NMR(300MHz)δTMS CDCℓ$_3$: 0.88(9H, m, -Me), 1.25(68H, m, -CH$_2$-), 2.17 - 2.57(7H, m, -COCH$_2$-, -COCH-), 3.85 - 4.24(5H, m, >CHOSEM, H-2, H-5, H$_2$-6), 4.58(1H, d, J = 11.7Hz, H-1), 4.81 - 4.94(2H, m, H-4, OH), 5.51(1H, t, J = 9.5Hz, H-3), 6.70(1H, d, J = 9.5Hz, >NH), 7.12 - 7.34(10H, m, Ph)

(Seventh step)

Using 86.2 mg of compound (7k) and proceeding in the same way as compound (A), compound (K) was obtained (74.0 mg, quant).

Melting point: 156 - 158 °C

IR (film) cm$^{-1}$: 3300, 2940, 2860, 1740, 1650, 1550

Example 12

2-Deoxy-3-O-{(2RS)-2-dodecylhexadecanoyl}-2-{(3R)-3-hydroxytetradecanamido)-6-O-{3-(methoxycarbonyl)propionyl}-4-O-phosphono-D-glucopyranose (compound L)

(Fourth step)

2-(Trimethylsilyl)ethyl 2-deoxy-3-O-{(2RS)-2-dodecylhexadecanoyl}-6-O-{3-(methoxycarbonyl)propionyl}-2-[(3R)-3-{2-(trimethylsilyl)ethoxymethoxy}tetradecanamido]-β-D-glucopyranoside (compound 51)

33 mg of succinic anhydride was dissolved in 5 mℓ of methanol. DMAP of catalytic quantity was added to the solution, and then the mixture was stirred for 30 minutes at room temperature. After the reaction mixture was concentrated in vacuo, succinic monomethyl ester was obtained. It was dissolved in 15 mℓ of dichloromethane. 230 mg of compound (4j) obtained in Third step of Example 10 and 76.9 mg of WSC•HCℓ were added to the solution, and the mixture was stirred for an hour at room temperature. The reaction mixture was directly chrometographed on silica gel (dichloromethane : methanol = 400 : 1) to be purified, thereby yielding an amorphous compound (51) (224.9 mg, yield 88.1%).

IR(Film)cm$^{-1}$: 3550, 3300, 2940, 2860, 1740, 1640, 1550, 860, 840

$^1$H-NMR(300MHz)δTMS CDCℓ$_3$: 0.00(18H, m, MeSi), 0.85(13H, m, CH$_2$TMS, -Me), 1.24(68H, m, -CH$_2$-), 2.21 - 2.69(7H, m, -COCH$_2$-, -COCH< ), 3.00(1H, d, J = 4.2Hz, OH), 3.48 - 3.95(9H, m, CH$_2$CH$_2$TMS, >CHOSEM, -OMe, H-2, H-4, H-5), 4.33(1H, d, J = 11.1Hz, H-6), 4.50(1H, dd, J = 12.0Hz, J = 3.6Hz, H-6), 4.61 - 4.73(3H, m, -OCH$_2$O-, H-1), 5.16(1H, t, J = 8.5Hz, H-3), 6.22(1H, d, J = 8.7Hz, >NH)

(Fifth step)

2-(Trimethylsilyl)ethyl 2-deoxy-4-O-diphenylphosphono-3-O-{(2RS)-2-dodecylhexadecanoyl}-6-O-{3-(methoxycarbonyl)propionyl}-2-[(3R)-3-{2-(trimethylsilyl)ethoxymethoxy}tetradecanamido]-β-D-glucopyranoside (compound 61)

Using 170 mg of compound (51) and proceeding in the same way as compound (6a), an amorphous compound (61) was obtained (190 mg, yield 93.1%).

In this procedure, pyridine was used as a solvent instead of pyridine-dichloromethane.

[α]$_D$: +1.83° (c = 0.87, CH$_2$Cℓ$_2$)

IR(Film)cm$^{-1}$: 3300, 2920, 2850, 1740, 1640, 1540, 940, 850, 830, 760 - 650

$^1$H-NMR(300MHz)δTMS CDCℓ$_3$: 0.00(18H, m, MeSi), 0.85(13H, m, CH$_2$TMS, -Me), 1.25(68H, m, -CH$_2$-), 2.27 - 2.63(7H, m, -COCH$_2$-, -COCH< ), 3.52 - 3.90(6H, m, CH$_2$CH$_2$TMS, >CHOSEM, H-2, H-4, H-5), 4.13(1H, dd, J = 12.1Hz, J = 4.4Hz, H-6), 4.37(1H, d, J = 10.2Hz, H-6), 4.65 - 4.80(3H, m, -OCH$_2$O-, H-1), 5.58(1H, t, J = 8.4Hz, H-3), 6.21(1H, d, J = 8.4Hz, >NH), 7.09 - 7.32(10H, m, Ph)

(Sixth step)

2-Deoxy-4-O-diphenylphosphono-3-O-{(2RS)-2-dodecylhexadecanoyl}-2-{(3R)-3-hydroxytetradecanamido}-6-O-{3-(methoxycarbonyl)propionyl}-D-glucopyranose(compound 71)

Using 190 mg of compound (61) and proceeding in the same way as compound (7a), an amorphous compound (71) was obtained (115.4 mg, yield 73.0%).

IR(Film)$cm^{-1}$: 3300, 2930, 2850, 1740, 1640, 1550, 950, 770 - 650

$^1$H-NMR(300MHz)δTMS CDCℓ$_3$: 0.88(9H, m, -Me), 1.25(68H, m, $-CH_2-$), 2.14 - 2.67(7H, m, $-COCH_2-$, -COCH< ), 3.61 - 3.72(4H, m, OMe, H-5), 3.95(1H, br, OH), 4.06(1H, dd, J=11.8Hz, J=4.4Hz, H-6), 4.22 - 4.37(3H, m, >CHOH, H-2, H-6), 4.69 - 4.81(2H, m, OH, H-4), 5.21(1H, br, H-1), 5.52(1H, t, J=9.3Hz, H-3), 6.33(1H, d, J=9.1Hz, >NH), 7.06 - 7.32(10H, m, Ph)

(Seventh step)

Using 115.4 mg of compound (71) and proceeding in the same way as compound (A), compound (L) was obtained (96.9 mg, quant).

In this procedure, ethanol was used as a solvent instead of methanol-ethanol.

Melting point: 153 - 155 °C

IR (film) $cm^{-1}$: 3300, 2940, 2860, 1740, 1650, 1550

Example 13

6-O-Acetyl-2-deoxy-2-{(3R)-3-hydroxytetradecanamido}-4-O-phosphono-3-O-{(3RS)-3-undecylheptadecanoyl}-D-glucopyranose (compound M)

(Second step)

2-(Trimethylsilyl)ethyl 2-deoxy-4,6-O-isopropylidene-2-[(3R)-3-{2-(trimethylsilyl)-ethoxymethoxy}tetradecanamido]-3-O-{(3RS)-3-undecylheptadecanoyl}-β-D-glucopyranoside (compound 3m)

Using 1.5g of compound (2j) and 1.41 mg of (3RS)-3-undecylheptadecanoic acid, and proceeding in the same way as compound (3a), a syrupy compound (3m) was obtained (2.40g, quant).

$^1$H-NMR(300MHz)δTMS CDCℓ$_3$: 0.00(18H, m, MeSi), 0.85(13H, m, $CH_2$TMS, -Me), 1.25(67H, m, $-CH_2-$, -CH< ), 2.13 - 2.35(4H, m, $-COCH_2-$), 3.31 - 4.00(10H, m, $CH_2CH_2$TMS, >CHOSEM, H-2, H-4, H-5, $H_2$-6), 4.54(1H, d, J=8.4Hz, H-1), 4.64 - 4.73(2H, 2d, J=12.4Hz, $-OCH_2O-$), 5.14(1H, t, J=9.7Hz, H-3), 6.20(1H, d, J=9.1Hz, >NH)

(Third step)

2-(Trimethylsilyl)ethyl 2-deoxy-2-[(3R)-3-{2-(trimethylsilyl)ethoxymethoxy}tetradecanamido]-3-O-{(3RS)-3-undecylheptadecanoyl}-β-D-glucopyranoside (compound 4m)

Using 2.39g of compound (3m) and proceeding in the same way as compound (4a), a syrupy compound (4m) was obtained (1.95g, yield 84.8%).

$^1$H-NMR(300MHz)δTMS CDCℓ$_3$: 0.00(18H, m, MeSi), 0.87(13H, m, $CH_2$TMS, -Me), 1.24(67H, m, $-CH_2-$, -CH< ), 2.24 - 2.35(4H, m, $-COCH_2-$), 2.95(1H, d, J=5.1Hz, OH), 3.45 - 3.96(8H, m, $CH_2CH_2$TMS, >CHOSEM, H-2, H-4, H-5, $H_2$-6), 4.63 - 4.72(3H, m, $-OCH_2O-$, H-1), 5.15(1H, t, J=9.5Hz, H-3), 6.28(1H, d, J=8.0Hz, >NH)

(Eighth step)

2-(Trimethylsilyl)ethyl 6-O-(tert)-butyldimethylsilyl-2-deoxy-2-[(3R)-3-{2-(trimethylsilyl)-ethoxymethoxy}tetradecanamido]-3-O-{(3RS)-3-undecylheptadecanoyl}-β-D-glucopyranoside (compound 8m)

Using 810 mg of compound (4m) and proceeding in the same way as compound (8j), a syrupy compound (8m) was obtained (780 mg, yield 87.6%).

$^1$H-NMR(300MHz)δTMS $CDC\ell_3$: 0.00(24H, m, MeSi), 0.90(22H, m, $CH_2$TMS, -Me), 1.25(67H, m, $-CH_2-$, -CH< ), 2.24 - 2.34(4H, m, $-COCH_2-$), 3.25(1H, d, J=2.6Hz, OH), 3.39 - 3.96(8H, m, $CH_2CH_2$TMS, > CHOSEM, H-2, H-4, H-5, $H_2$-6), 4.52(1H, d, J=8.2Hz, H-1), 4.63 - 4.73(2H, 2d, J=9.0Hz, $-OCH_2O-$), 5.09(1H, t, J=9.0Hz, H-3), 6.18(1H, d, J=9.0Hz, > NH)

(Ninth step)

2-(Trimethylsilyl)ethyl 6-O-(tert)-butyldimethylsilyl-2-deoxy-4-O-diphenylphosphono-2-[(3R)-3-{2-(trimethylsilyl)ethoxymethoxy}tetradecanamido]-3-O-{(3RS)-3-undecylheptadecanoyl}-β-D-glucopyranoside (compound 9m)

Using 720 mg of compound (8m) and proceeding in the same way as compound (9j), an amorphous compound (9m) was obtained (830 mg, yield 95.9%).

$^1$H-NMR(300MHz)δTMS $CDC\ell_3$: 0.00(24H, m, MeSi), 0.85(22H, m, $CH_2$TMS, -Me), 1.25(67H, m, $-CH_2-$, -CH< ), 2.11 - 2.35(4H, m, $-COCH_2-$), 3.21 - 3.98(8H, m, $CH_2CH_2$TMS, > CHOSEM, H-2, H-4, H-5, $H_2$-6), 4.52 - 4.74(1H, m, $-OCH_2O-$, H-1), 5.48(1H, t, J=9.2Hz, H-3), 6.15(1H, d, J=8.9Hz, > NH), 7.13(10H, m, Ph)

(Tenth step)

2-(Trimethylsilyl)ethyl 2-deoxy-4-O-diphenylphosphono-2-[(3R)-3-{2-(trimethylsilyl)ethoxymethoxy}tetradecanamido]-3-O-{(3RS)-3-undecylheptadecanoyl}-β-D-glucopyranoside (compound 10m)

Using 720 mg of compound (9m) and proceeding in the same way as compound (10j), an amorphous compound (10m) was obtained (370 mg, yield 51.1%).

$^1$H-NMR(300MHz)δTMS $CDC\ell_3$: 0.00(18H, m, MeSi), 0.88(13H, m, $CH_2$TMS, -Me), 1.25(67H, m, $-CH_2-$, -CH< ), 2.08 - 2.34(4H, m, $-COCH_2-$), 3.20 - 3.97(8H, m, $CH_2CH_2$TMS, > CHOSEM, H-2, H-4, H-5, $H_2$-6), 4.65 - 4.84(3H, m, $-OCH_2O-$, H-1), 5.63(1H, t, J=9.3Hz, H-3), 6.26(1H, d, J=8.4Hz, > NH), 7.14 - 7.36(10H, m, Ph)

(Eleventh step)

2-(Trimethylsilyl)ethyl 6-O-acetyl-2-deoxy-4-O-diphenylphosphono-2-[(3R)-3-{2-(trimethylsilyl)ethoxymethoxy}tetradecanamido]-3-O-{(3RS)-3-undecylheptadecanoyl}-β-D-glucopyranoside (compound 6m)

Using 168 mg of compound (10m) and proceeding in the same way as compound (6j), an amorphous compound (6m) was obtained (160 mg, yield 91.1%).

$^1$H-NMR(300MHz)δTMS $CDC\ell_3$: 0.00(18H, m, MeSi), 0.88(13H, m, $CH_2$TMS, -Me), 1.25(67H, m, $-CH_2-$, -CH< ), 1.98(3H, s, $-COCH_3$), 2.12 - 2.33(4H, m, $-COCH_2-$), 3.52 - 3.91(6H, m, $CH_2CH_2$TMS, > CHOSEM, H-2, H-4, H-5), 4.08(1H, dd, J=12.1Hz, H-6), 4.31(1H, d, J=10.2Hz, H-6), 4.64 - 4.81(3H, m, $-OCH_2O-$, H-1), 5.56(1H, t, J=9.0Hz, H-3), 6.23(1H, d, J=8.4Hz, > NH), 7.12 - 7.33(10H, m, Ph)

(Sixth step)

6-O-Acetyl-2-deoxy-4-O-dipheynlphosphono-2-{(3R)-3-hydroxytetradecanamido}-3-O-{(3RS)-3-undecylheptadecanoyl}-β-D-glucopyranoside (compound 7m)

Using 150 mg of compound (6m) and proceeding in the same way as compound (7a), an amorphous compound (7m) was obtained (113.3 mg, yield 91.7%).

$^1$H-NMR(300MHz)δTMS $CDC\ell_3$: 0.87(9H, m, -Me), 1.25(67H, m, $-CH_2-$, -CH< ), 2.00(3H, s, $-COCH_3$), 2.10 - 2.25(4H, m, $-COCH_2-$), 3.61(1H, br, OH), 3.96 - 4.28(4H, m, > CHOSEM, H-2, H-4, H-5), 4.77 - 4.84-(2H, m, H-4, OH), 5.21(1H, br, H-1), 5.48(1H, t, J=9.3Hz, H-3), 6.34(1H, d, J=9.1Hz, > NH), 7.11 - 7.34-(10H, m, Ph)

(Seventh step)

Using 111.7 mg of compound (7m) and proceeding in the same way as compound (A), objective compound (M) was obtained (95.9 mg, quant).

In this procedure, ethanol was used as a solvent instead of methanol-ethanol.

Melting point: 172 - 174 °C

Example 14

2-Deoxy-6-O-(3-carboxypropionyl)-2-{(3R)-3-hydroxytetradecanamido}-4-O-phosphono-3-O-{(3RS)-3-undecylheptadecanoyl}-D-glucopyranose (compound N)

(Eleventh step)

2-(Trimethylsilyl)ethyl 2-deoxy-4-O-diphenylphosphono-6-O-(3-carboxypropionyl)-2-[(3R)-3-{2-(trimethylsilyl)ethoxymethoxy}tetradecanamido]-3-O-{(3RS)-3-undecylheptadecanoyl}-β-D-glucopyranoside (compound 6n)

Using 115 mg of compound (10m) obtained in Tenth step of Example 13 and proceeding in the same way as compound (6k), an amorphous compound (6n) was obtained (172 mg, yield 91.5%).

$^1$H-NMR(300MHz)δTMS CDCℓ$_3$: 0.00(18H, m, MeSi), 0.88(13H, m, $CH_2$TMS, -Me), 1.25(67H, m, -$CH_2$-, -CH< ), 2.11 - 2.63(8H, m, -$COCH_2$-), 3.48 - 3.90(6H, m, -$CH_2CH_2$TMS, >CHOSEM, H-2, H-4, H-5), 4.03-(1H, dd, J = 12.0Hz, J = 3.8Hz, H-6), 4.38(1H, d, J = 10.4Hz, H-6), 4.64 - 4.79(3H, m, -$OCH_2O$-, H-1), 5.53(1H, t, J = 9.0Hz, H-3), 6.30(1H, d, J = 8.6Hz, >NH), 7.11 - 7.33(10H, m, Ph)

(Sixth step)

2-Deoxy-4-O-diphenylphosphono-6-O-(3-carboxypropionyl)-2-{(3R)-3-hydroxytetradecanamido}-3-O-{(3RS)-3-undecylheptadecanoyl}-D-glucopyranoside (compound 7n)

Using 170.2 mg of compound (6n) and proceeding in the same way as compound (7a), an amorphous compound (7n) was obtained (126.9 mg, yield 89.7%).

$^1$H-NMR(300MHz)δTMS CDCℓ$_3$: 0.88(9H, m, -Me), 1.25(67H, m, -$CH_2$-, -CH< ), 2.05 - 2.61(8H, m, -$COCH_2$-), 3.76 - 4.22(4H, m, >CHOSEM, H-2, H-5, H-6), 4.59(1H, d, J = 11.7Hz, H-1), 4.81(1H, q, J = 9.5Hz, H-4), 4.94(1H, br, OH), 5.48(1H, t, J = 9.5Hz, H-3), 6.57(1H, d, J = 9.3Hz, >NH), 7.10 - 7.34(10H, m, Ph)

(Seventh step)

Using 88.9 mg of compound (7n) and proceeding in the same way as compound (A), objective compound (N) was obtained (81.7 mg, quant).

In this procedure, ethanol was used as a solvent instead of methanol-ethanol.

Melting point: 156 - 158 °C

Example 15

2-Deoxy-2-{(3R)-3-hydroxytetradecanamido}-6-O-{3-(methoxycarbonyl)propionyl}-4-O-phosphono-3-O-{-(3RS)-3undecylheptadecanoyl}-D-glucopyranose (compound O)

(Fourth step)

2-(Trimethylsilyl)ethyl 2-deoxy-6-O-{3-(methoxycarbonyl)propionyl}-2-[(3R)-3-{2-(trimethylsilyl)-ethoxymethoxy}tetradecanamido]-3-O-{(3RS)-3-undecylheptadecanoyl}-β-D-glucopyranoside (compound 5o)

Using 450 mg of compound (4m) obtained in Third step of Example 13 and proceeding in the same way as compound (51), an amorphous compound (5o) was obtained (410 mg, yield 82.1%).

$^1$H-NMR(300MHz)δTMS CDCℓ$_3$: 0.00(18H, m, MeSi), 0.85(13H, m, $CH_2$TMS, -Me), 1.24(67H, m, -$CH_2$-,

-CH< ), 2.24 - 2.70(8H, m, $-COCH_2-$), 2.98(1H, d, J=4.4Hz, OH), 3.48 - 3.96(9H, m, $CH_2CH_2TMS$, >CHOSEM, -OMe, H-2, H-4, H-5), 4.33(1H, d, J=11.1Hz, H-6), 4.50(1H, dd, J=11.9Hz, J=3.6Hz, H-6), 4.61 - 4.72(3H, m, $-OCH_2O-$, H-1), 5.13(1H, t, J=8.4Hz, H-3), 6.19(1H, d, J=8.7Hz, >NH)

(Fifth step)

2-(Trimethylsilyl)ethyl 2-deoxy-4-O-diphenylphosphono-6-O-{3-(methoxycarbonyl)propionyl}-2-[(3R)-3-{2-(trimethylsilyl)ethoxymethoxy}tetracanamido]-3-O-{(3RS)-3-undecylheptadecanoyl}-β-D-glucopyranoside (compound 6o)

Using 300 mg of compound (5o) and proceeding in the same way as compound (6a), an amorphous compound (6o) was obtained (260 mg, yield 72.1%).

$^1$H-NMR(300MHz)δTMS CDCℓ$_3$: 0.00(18H, m, MeSi), 0.85(13H, m, $CH_2TMS$, -Me), 1.25(67H, m, $-CH_2-$, -CH< ), 2.11 - 2.64(8H, m, $-COCH_2-$), 3.49 - 3.91(9H, m, $CH_2CH_2TMS$, >CHOSEM, -OMe, H-2, H-4, H-5), 4.10(1H, dd, J=12.1Hz, J=4.5Hz, H-6), 4.64 - 4.73(2H, 2d, J=9.2Hz, $-OCH_2O-$), 4.78(1H, d, J=8.0Hz, H-1), 5.55(1H, t, J=9.1Hz, H-3), 6.22(1H, d, J=8.7Hz, >NH), 7.11 - 7.33(10H, m, Ph)

(Sixth step)

2-Deoxy-4-O-diphenylphosphono-2-{(3R)-3-hydroxytetradecanamido}-6-O-{3-(methoxycarbonyl)propionyl}-3-O-{(3RS)-3-undecylheptadecanoyl}-D-glucopyranoside (compound 7o)

Using 190 mg of compound (6o) and proceeding in the same way as compound (7a), an amorphous compound (7o) was obtained (145.9 mg, yield 94.6%).

$^1$H-NMR(300MHz)δTMS CDCℓ$_3$: 0.88(9H, m, -Me), 1.25(67H, m, $-CH_2-$, -CH< ), 2.09 - 2.59(8H, m, $-COCH_2-$), 3.60 - 3.70(4H, m, OMe, H-5), 3.94(1H, br, OH), 4.01(1H, dd, J=12.4Hz, J=4.6Hz, H-6), 4.19 - 4.33(3H, m, >CHOSEM, H-2, H-6), 4.67 - 4.78(2H, m, OH, H-4), 5.22(1H, br, H-1), 5.47(1H, t, J=9.3Hz, H-3), 6.30(1H, d, J=9.0Hz, >NH), 7.12 - 7.34(10H, m, Ph)

(Seventh step)

Using 149.5 mg of compound (7o) and proceeding in the same way as compound (A), objective compound (O) was obtained (128.3 mg, quant).

In this procedure, ethanol was used as a solvent instead of the methanol-ethanol.

Melting point: 154 - 156 °C.

## Claims

**1.** 6-Substituted lipid A analogues expressed in Formula [I]:

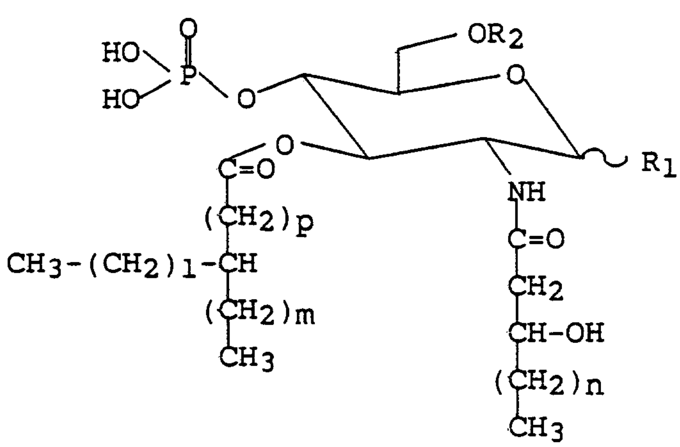

[I]

wherein $R_1$ is a hydrogen atom or a hydroxyl group, $R_2$ is $-C(O)R_3$, $R_3$ is a lower alkyl group of straight chain or branch which may be replaced by carboxyl group or lower alkoxycarbonyl group, 1 is an

integer of 5 to 17, m is an integer of 7 to 19, n is an integer of 4 to 16, and p is either 0 or 1.

# INTERNATIONAL SEARCH REPORT

International Application No PCT/JP91/01628

**I. CLASSIFICATION OF SUBJECT MATTER** (if several classification symbols apply, indicate all) [6]

According to International Patent Classification (IPC) or to both National Classification and IPC

Int. $Cl^5$ C07F9/655, C07H13/06//A61K31/70, A61K31/665

**II. FIELDS SEARCHED**

Minimum Documentation Searched [7]

| Classification System | Classification Symbols |
|---|---|
| IPC | C07F9/655, C07H13/06, A61K31/70, A61K31/665 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 03-106894 (Japan Tobacco Inc.), May 7, 1991 (07. 05. 91) & EP, A1, 444208 & WO, A1, 91/4259 | 1 |
| A | JP, A, 03-264594 (Japan Tobacco Inc.), November 25, 1991 (25. 11. 91), (Family: none) | 1 |
| A | JP, A, 02-25494 (Japan Tobacco Inc.), January 26, 1990 (26. 01. 90), (Family: none) | 1 |
| A | JP, A, 01-146892 (Toho Pharmaceutical Co., Ltd., Akira Hasegawa), June 8, 1989 (08. 06. 89), (Family: none) | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 14, 1992 (14. 02. 92) | March 3, 1992 (03. 03. 92) |
| **International Searching Authority** | **Signature of Authorized Officer** |
| Japanese Patent Office | |